# EUROPEAN PATENT SPECIFICATION

(11) **EP 4 382 152 B1**
(45) Date of publication and mention of the grant of the patent: **25.02.2026**
(21) Application number: 22852739.6
(22) Date of filing: 29.06.2022
(51) Int. Cl.: A61M 11/00, A61M 15/08, A61M 15/00, A61M 16/06, B05B 1/14, B05B 1/34

(54) **SPRAYER**
SPRÜHGERÄT
PULVÉRISATEUR

(30) Priority: 02.08.2021 JP 2021126675
(43) Date of publication of application: 12.06.2024
(73) Proprietor: Shinko Chemical Co., Ltd., Kanazawa-shi Ishikawa 920-0346 (JP)
(72) Inventor: DOI, Atsushi, Kanazawa-shi Ishikawa 920-0346 (JP)
(74) Representative: HGF
(86) International application number: PCT/JP2022/026018
(87) International publication number: WO 2023/013313

(56) References cited:
- WO-A2-2015/018758
- CN-A- 1 279 621
- JP-A- 2007 105 365
- JP-A- 2013 132 452
- JP-A- 2013 132 452
- JP-A- H11 114 065

## Description

### BACKGROUND OF THE DISCLOSURE

### Field of the Disclosure

The present disclosure relates to a sprayer for spraying liquid into nostrils.

### Description of the Related Art

This type of sprayer is described in, for example, Patent Document 1. Patent Document 1 describes a sprayer for nostrils connectable to a container containing liquid to discharge the liquid from two nozzle cylinders into two nostrils at the same time. The sprayer for nostrils includes a button, a push portion attached to the button as a cap, and a tubular stem connected to the push portion to allow the liquid to flow through. The button includes a flat plate, the two nozzle cylinders extending upward on the plate, and a downward cylinder extending downward from the plate. The push portion is fitted to the downward cylinder in the button. This defines a space between the plate in the button and the top of the push portion to allow passage of the liquid being sprayed. The plate in the button has two connection holes connecting the space and the internal spaces of the nozzle cylinders to allow fluid passage. In other words, the two connection holes are located inward from the downward cylinder in the button as viewed in the axial direction of the nozzle cylinders. The liquid is supplied to the space described above through the stem, enters the nozzle cylinders through the connection holes, and is sprayed from spray holes in the distal ends of the nozzle cylinders.

Patent Document 1: Japanese Unexamined Patent Application Publication No. 2007-105365

The following document is also known in the art, namely JP 2013 132452 A.

### BRIEF SUMMARY OF THE DISCLOSURE

The invention is defined in the appended claims. A sprayer including one stem and two nozzle cylinders, similarly to the sprayer for nostrils described above, is substantially Y-shaped as viewed in the radial direction of the nozzle cylinders. The sprayer with this shape typically includes a component for the nozzle cylinders (e.g., the button) and a component for the container (e.g., the push portion) that are formed separately and combined together for ease of manufacture. In this structure, liquid may leak through a contact between the components when the internal pressure of flow channels increases during spraying.

One or more aspects of the present disclosure are directed to a sprayer that reduces liquid leakage.

A sprayer according to one aspect of the present disclosure includes a nozzle insertable into a nostril and an adapter to connect the nozzle and a container containing liquid.

The nozzle includes at least one sub-nozzle having a distal end and a basal end. The distal end has a nozzle bore and is insertable into the nostril.

The adapter includes a coupling pipe, two cores, and receivers being U-shaped and annular.

The coupling pipe has a coupling end connectable to the container to allow fluid passage.

Each of the two cores extends in an axial direction in which the coupling pipe extends and has an axis different from an axis of the coupling pipe in a radial direction intersecting with the axial direction. The two cores are adjacent to each other.

Each of the receivers is at an outer side surface and a basal end of a corresponding core of the two cores and is open toward a distal end of the corresponding core.

Each of the receivers has a hole connecting with the coupling pipe to allow fluid passage.

The nozzle is attached to the adapter with a core of the two cores received in an internal space of the at least one sub-nozzle and the basal end of the at least one sub-nozzle received in a corresponding receiver of the receivers.

The at least one sub-nozzle and the core define a core path in between. The core path connects with the coupling pipe through the hole to allow fluid passage.

The core path is at least a part of a flow channel extending from the hole to the nozzle bore.

In an attachment state of the nozzle attached to the adapter, an outer surface of the at least one sub-nozzle and a facing surface of the corresponding receiver facing the outer surface are in contact with each other in a liquid tight manner and divide the core path from outside the core path.

In the attachment state, a contact between the outer surface of the at least one sub-nozzle and the facing surface of the corresponding receiver is in a shape of a perfect circle or substantially a perfect circle in a cross section intersecting with the axial direction.

The sprayer according to the above aspect of the present disclosure reduces liquid leakage.

### BRIEF DESCRIPTION OF THE SEVERAL VIEWS OF THE DRAWINGS

Fig. 1 is a perspective view of a sprayer 1 according to an embodiment of the present disclosure and a syringe 9.
Fig. 2 is an exploded perspective view of the sprayer 1 and the syringe 9 in Fig. 1.
Fig. 3 is a perspective view of the sprayer 1 in Fig. 1.
Fig. 4 is a plan view of the sprayer 1 in Fig. 1.
Fig. 5 is an end view of the sprayer 1 in Fig. 4 taken along line V-V.
Fig. 6 is a bottom view of a nozzle 2 in Fig. 2.
Fig. 7 is a perspective view of a rod 3 in Fig. 2.
Fig. 8 is a perspective view of an adapter 4 in Fig. 2.
Fig. 9 is a plan view of the adapter 4 in Fig. 2.
Fig. 10 is a partially enlarged view of a receiver 45 in Fig. 5.
Fig. 11 is a partially enlarged view of area Z1 in Fig. 5.
Fig. 12 is a side view of a stopper 5 in Fig. 2.
Fig. 13 is an end view of the sprayer 1 in Fig. 4 taken along line V-V.
Fig. 14 is a cross-sectional view of the sprayer 1 in Fig. 13 taken along line XIV-XIV.
Fig. 15 is a cross-sectional view of the sprayer 1 in Fig. 13 taken along line XV-XV.
Fig. 16 is a cross-sectional view of the sprayer 1 in Fig. 13 taken along line XVI-XVI.
Fig. 17 is a cross-sectional view of the sprayer 1 in Fig. 13 taken along line XVII-XVII.
Fig. 18 is a cross-sectional view of the sprayer 1 in Fig. 13 taken along line XVIII-XVIII.
Fig. 19 is a partially enlarged view of a sprayer 1 according to a first modification of the embodiment of the present disclosure corresponding to Fig. 11.
Fig. 20 is a partially enlarged view of a sprayer 1 according to a second modification of the embodiment of the present disclosure corresponding to Fig. 11.
Fig. 21 is a perspective view of a sprayer 1A according to a third modification of the embodiment of the present disclosure.
Fig. 22 is a perspective view of a chip 28 in the sprayer 1A in Fig. 21.
Fig. 23 is a cross-sectional view of the sprayer 1A in Fig. 21 taken along line XXIII-XXIII.
Fig. 24 is a cross-sectional view of the sprayer 1A in Fig. 23 taken along line XXIV-XXIV.
Fig. 25 is a cross-sectional view of the sprayer 1A in Fig. 23 taken along line XXV-XXV

### DETAILED DESCRIPTION OF THE DISCLOSURE

A sprayer according to a first aspect of the present disclosure includes a nozzle insertable into a nostril and an adapter to connect the nozzle and a container containing liquid.

The nozzle includes at least one sub-nozzle having a distal end and a basal end. The distal end has a nozzle bore and is insertable into the nostril.

The adapter includes a coupling pipe, two cores, and receivers being U-shaped and annular.

The coupling pipe has a coupling end connectable to the container to allow fluid passage.

Each of the two cores extends in an axial direction in which the coupling pipe extends and has an axis different from an axis of the coupling pipe in a radial direction intersecting with the axial direction. The two cores are adjacent to each other.

Each of the receivers is at an outer side surface and a basal end of a corresponding core of the two cores and is open toward a distal end of the corresponding core.

Each of the receivers has a hole connecting with the coupling pipe to allow fluid passage.

The nozzle is attached to the adapter with a core of the two cores received in an internal space of the at least one sub-nozzle and the basal end of the at least one sub-nozzle received in a corresponding receiver of the receivers.

The at least one sub-nozzle and the core define a core path in between. The core path connects with the coupling pipe through the hole to allow fluid passage.

The core path is at least a part of a flow channel extending from the hole to the nozzle bore.

In an attachment state of the nozzle attached to the adapter, an outer surface of the at least one sub-nozzle and a facing surface of the corresponding receiver facing the outer surface are in contact with each other in a liquid tight manner and divide the core path from outside the core path.

In the attachment state, a contact between the outer surface of the at least one sub-nozzle and the facing surface of the corresponding receiver is in a shape of a perfect circle or substantially a perfect circle in a cross section intersecting with the axial direction.

A sprayer according to a second aspect of the present disclosure is the sprayer according to the first aspect, in which
the contact is between the coupling pipe and the nozzle bore in the axial direction.

A sprayer according to a third aspect of the present disclosure is the sprayer according to the first aspect or the second aspect, in which
in the attachment state, the facing surface of each of the receivers is inclined away from the outer side surface of the corresponding core toward an opening in the receiver in the axial direction, and
the outer surface of the at least one sub-nozzle and the facing surface of the corresponding receiver are linearly in contact with each other at the contact.

A sprayer according to a fourth aspect of the present disclosure is the sprayer according to any one of the first to third aspects, in which
each of the receivers includes an annular projection protruding outward from an outer surface being a surface opposite to the facing surface,
the nozzle includes a protrusion protruding toward the facing surface of a corresponding receiver of the receivers in the attachment state, and
in the attachment state, the protrusion on the nozzle is located farther from the nozzle bore than the annular projection on each of the receivers in the axial direction and is in contact with the annular projection on the corresponding receiver.

A sprayer according to a fifth aspect of the present disclosure is the sprayer according to any one of the first to fourth aspects, in which
the nozzle includes two sub-nozzles,
in the attachment state, each of the cores is received in the internal space of a corresponding sub-nozzle of the two sub-nozzles, and
in the attachment state, the contact between the outer surface of each of the two sub-nozzles and the facing surface of the corresponding receiver is in a shape of a perfect circle or substantially a perfect circle in a cross section intersecting with the axial direction.

A sprayer according to a sixth aspect of the present disclosure is the sprayer according to the fifth aspect, in which
each of the two cores has an edge defined by the basal end of the core in a plan view of the adapter.

A sprayer according to a seventh aspect of the present disclosure is the sprayer according to the fifth aspect or the sixth aspect, in which
each of the two sub-nozzles has, in the internal space of the sub-nozzle and between the distal end of the sub-nozzle and the distal end of a corresponding core of the two cores, a chamber being at least a part of the flow channel, and
the chamber has a larger channel cross section than the core path in the flow channel.

A sprayer according to an eighth aspect of the present disclosure is the sprayer according to the seventh aspect, further including
rods each in the internal space of a corresponding sub-nozzle of the two sub-nozzles, each of the rods being between the distal end of the corresponding sub-nozzle and the distal end of the corresponding core,
in which the chamber is between each of the rods and the distal end of the corresponding sub-nozzle, and
each of the rods and an inner side surface of the corresponding sub-nozzle define a rod path in between.

A sprayer according to a ninth aspect of the present disclosure is the sprayer according to the eighth aspect, in which
the rod path has a smaller channel cross section than the chamber in the flow channel.

A sprayer according to a tenth aspect of the present disclosure is the sprayer according to the eighth aspect or the ninth aspect, in which
each of the rods is in contact with the distal end of the corresponding sub-nozzle, and
each of the rods and the distal end of the corresponding sub-nozzle define a guide path in between, and the guide path extends in a direction intersecting with the axial direction and connects the rod path and the nozzle bore to allow fluid passage.

A sprayer according to an eleventh aspect of the present disclosure is the sprayer according to any one of the first to tenth aspects, in which
the core path has a smaller channel cross section than the hole in the flow channel.

A sprayer according to a twelfth aspect of the present disclosure is the sprayer according to any one of the first to eleventh aspects, in which
each of the receivers includes a portion overlapping the coupling pipe and a portion not overlapping the coupling pipe in a plan view of the adapter.

A sprayer according to a thirteenth aspect of the present disclosure is the sprayer according to any one of the first to twelfth aspects, in which
the coupling pipe includes
a large cross-sectional portion nearer the coupling end than the hole in the axial direction, and
a small cross-sectional portion nearer the coupling end than the large cross-sectional portion in the axial direction and having a smaller cross section than the large cross-sectional portion in the axial direction,
the sprayer further includes a stopper in an internal space of the coupling pipe,
the stopper is in contact with an inner surface of the coupling pipe in a liquid tight manner in the small cross-sectional portion and defines a clearance with the inner surface of the coupling pipe in the large cross-sectional portion, and
the stopper slides from the small cross-sectional portion to the large cross-sectional portion when pressed by the liquid in a direction from the connection end in the axial direction.

A sprayer according to a fourteenth aspect of the present disclosure is the sprayer according to the thirteenth aspect, in which
the coupling pipe includes an expanded portion between the large cross-sectional portion and the small cross-sectional portion, and the expanded portion has a cross section gradually increasing toward the large cross-sectional portion in the axial direction.

A sprayer according to a fifteenth aspect of the present disclosure is the sprayer according to the fourteenth aspect, in which
the coupling pipe includes a reduced portion between the expanded portion and the small cross-sectional portion, and the reduced portion has a cross section gradually decreasing away from the small cross-sectional portion in the axial direction.

A sprayer according to a sixteenth aspect of the present disclosure is the sprayer according to any one of the first to fifteenth aspects, in which
each of the nozzle and the adapter is symmetric about the axis of the coupling pipe.

A sprayer according to an embodiment of the present disclosure will now be described with reference to the drawings. In the present embodiment, the same reference numerals denote the same components. Such components will not be described.

### (1. Structure)

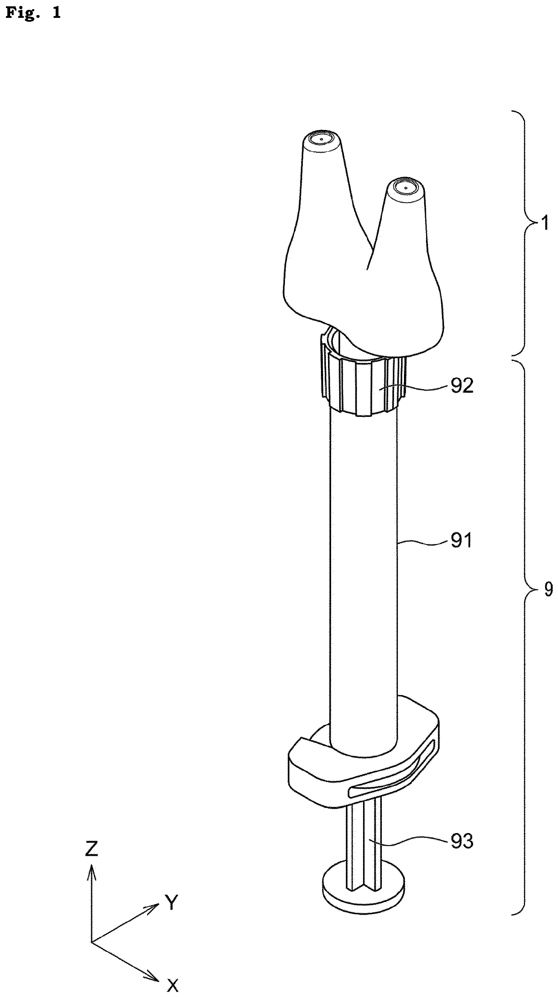

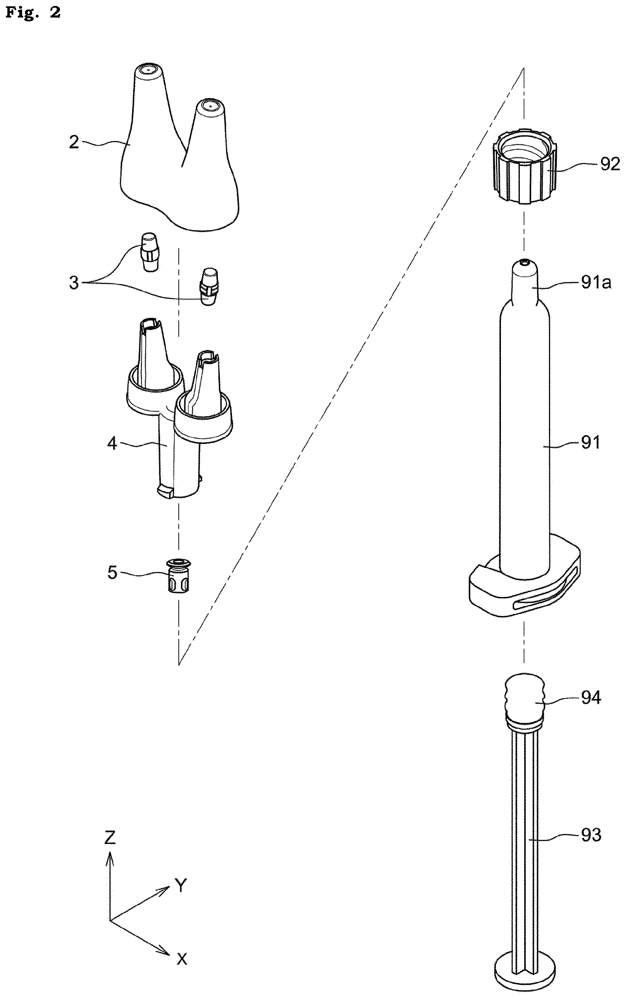

Fig. 1 is a perspective view of a sprayer 1 according to an embodiment of the present disclosure and a syringe 9. Fig. 2 is an exploded perspective view of the sprayer 1 and the syringe 9 in Fig. 1. For ease of explanation, Figs. 1 and 2 as well as Figs. 3 to 6, 8 to 21, and 23 to 25 (referred to later) show X-axis, Y-axis, and Z-axis that are orthogonal to one another. The positive direction along Z-axis is herein also referred to as up, and the negative direction along Z-axis as down with the sprayer in normal use. However, these directional terms do not limit, for example, the use state of the sprayer according to one or more embodiments of the present disclosure.

As shown in Fig. 1, the sprayer 1 is connectable to the syringe 9 and is used to spray liquid into two nostrils at the same time. The syringe 9 is an example of a container in an aspect of the present disclosure. As shown in Fig. 2, the sprayer 1 includes a nozzle 2 insertable into nostrils, rods 3 in the internal space of the nozzle 2, an adapter 4 attached to the nozzle 2, and a stopper 5 in an internal space of the adapter 4.

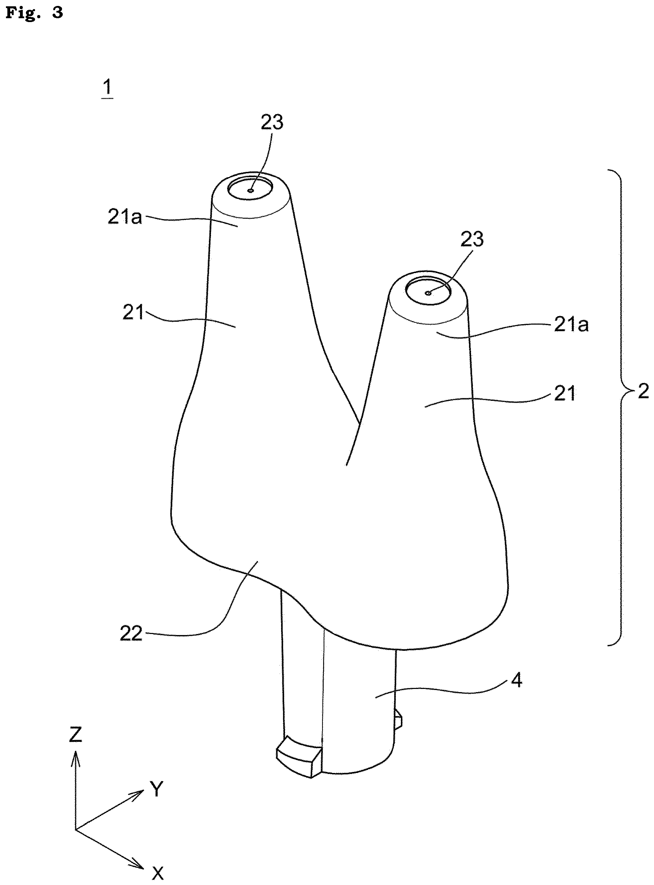

Fig. 3 is a perspective view of the sprayer 1 in Fig. 1. As shown in Fig. 3, the nozzle 2 includes two sub-nozzles 21 adjacent to each other and extending along Z-axis and a base 22 connecting the two sub-nozzles 21. The nozzle 2 is formed from, for example, a synthetic resin material such as polypropylene, high-density polyethylene, low-density polyethylene, linear low-density polyethylene, polyacetal, or polybutylene terephthalate. The nozzle 2 may be formed as one component by injection molding.

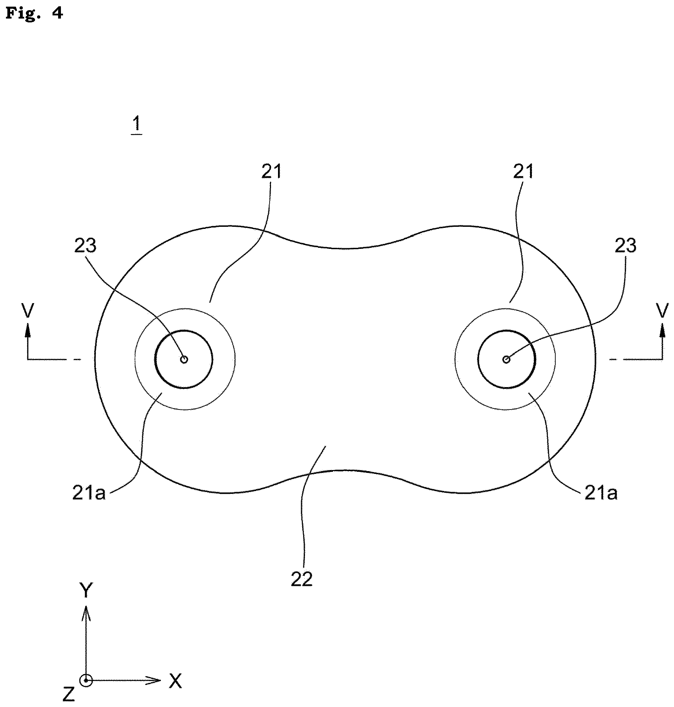

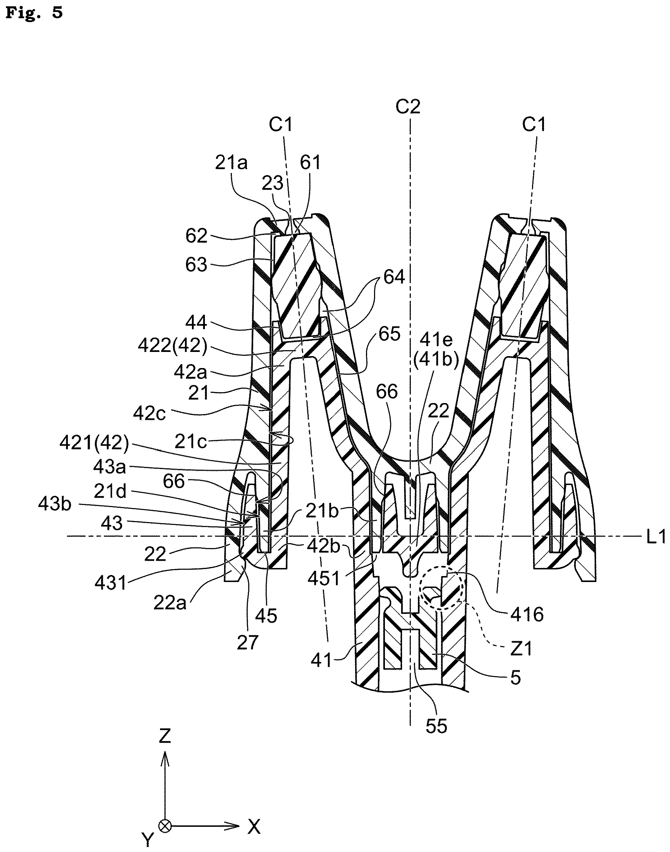

Fig. 4 is a plan view of the sprayer 1 in Fig. 1. Fig. 5 is an end view of the sprayer 1 in Fig. 4 taken along line V-V. Fig. 5 shows two imaginary axes C1 and one imaginary axis C2. Each imaginary axis C1 extends through the center of the corresponding sub-nozzle 21 in an XY cross section. The imaginary axis C2 extends through the center of a coupling pipe 41 (described later) in the adapter 4 in an XY cross section. In the example shown in Fig. 5, the sprayer 1 is symmetric about the imaginary axis C2. In other words, the two imaginary axes C1 extend symmetrically about the imaginary axis C2. In Fig. 5, the components located leftward from the imaginary axis C2 alone are given reference numerals.

As shown in Fig. 5, each sub-nozzle 21 is cylindrical and has a distal end 21a insertable into a nostril and a basal end 21b opposite to the distal end 21a. The distal end 21a of the sub-nozzle 21 is closed except at a nozzle bore 23 (described later). The basal end 21b of the sub-nozzle 21 is open.

Each sub-nozzle 21 extends in the axial direction of the corresponding imaginary axis C1. In other words, the imaginary axes C1 extend along Z-axis. For example, each imaginary axis C1 may be inclined at an angle of 0 to 15° with respect to the imaginary axis C2 away from the imaginary axis C2 in the positive direction along Z-axis. In the example shown in Figs. 3 and 5, each sub-nozzle 21 has the diameter gradually decreasing toward its distal end 21a along the corresponding imaginary axis C1.

As shown in Figs. 3 to 5, each sub-nozzle 21 has, at the center of the distal end 21a, a nozzle bore 23 for spraying liquid. In the example shown in Figs. 3 to 5, each nozzle bore 23 is circular and has a diameter of 300 µm when the sub-nozzles 21 are viewed in a direction along the imaginary axes C1. The shape of the nozzle bore 23 is not limited to the above example. The nozzle bore 23 may be circular and have a diameter of, for example, 200 to 600 µm when the sub-nozzles 21 are viewed in the direction along the imaginary axes C1.

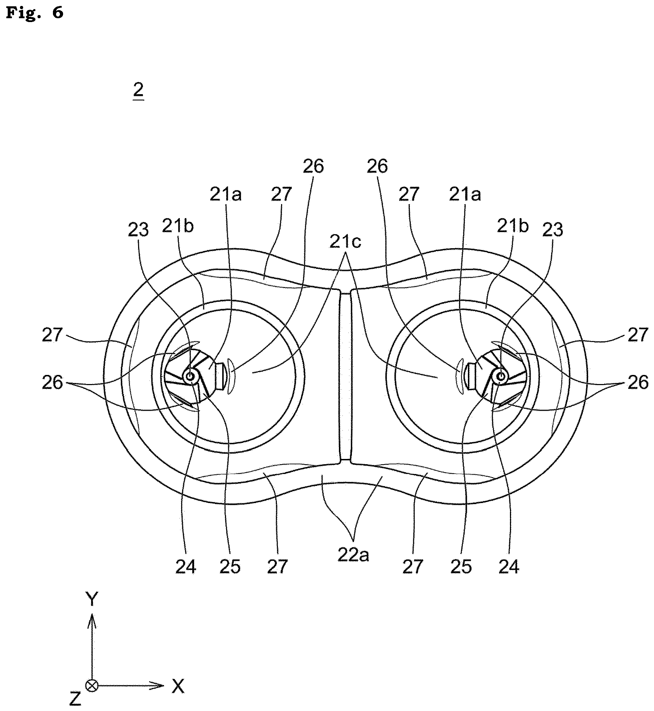

Fig. 6 is a bottom view of the nozzle 2 in Fig. 2. As shown in Fig. 6, each sub-nozzle 21 has, on the inner surface of its distal end 21a, a recess 24 at the center of the inner surface and three grooves 25 connecting with the recess 24. The recess 24 is exposed outside the nozzle 2 through the corresponding nozzle bore 23. The recess 24 is substantially truncated conical and tapers from the inner surface of the distal end 21a of the corresponding sub-nozzle 21 toward the nozzle bore 23. The recess 24 has an area in a cross section orthogonal to the corresponding imaginary axis C1 larger than the area of the open face of the nozzle bore 23.

Each groove 25 extends in the tangential direction of the circular recess 24 from the rim of the distal end 21a of the corresponding sub-nozzle 21 in a bottom view of the sub-nozzle 21 viewed from below along Z-axis. In the example shown in Fig. 6, the three grooves 25 are at intervals of 120° in the circumferential direction of the corresponding imaginary axis C1. The number of grooves 25 and the shape of the grooves 25 in a bottom view of the sub-nozzle 21 are not limited to this example. For example, two grooves 25 or four or more grooves 25 may be at equal intervals in the circumferential direction of the imaginary axis C1.

As shown in Fig. 5, each sub-nozzle 21 has a radially inner side surface about the corresponding imaginary axis C1. The radially inner side surface is an inner side surface 21c. As shown in Fig. 6, each sub-nozzle 21 includes, on its inner side surface 21c, projections 26 protruding toward the corresponding imaginary axis C1. The projections 26 are used to attach the rods 3 to the sub-nozzles 21. In the example shown in Fig. 6, three projections 26 are at intervals of 120° in the circumferential direction of the corresponding imaginary axis C1. The number of projections 26 and the shape of the projections 26 are not limited to this example. For example, each sub-nozzle 21 may include two or fewer or four or more projections 26.

As shown in Fig. 3, the base 22 in the nozzle 2 covers the basal ends 21b of the two sub-nozzles 21. As shown in Fig. 5, the base 22 includes portions continuous with the circumferences of the sub-nozzles 21 and integral with each other between the two sub-nozzles 21. As shown in Fig. 6, the base 22 has two lower edges 22a each surrounding the basal end 21b of the corresponding sub-nozzle 21 in a bottom view of the nozzle 2.

Each lower edge 22a includes three protrusions 27 protruding inward toward the basal end 21b of the corresponding sub-nozzle 21. The base 22 thus includes a total of six protrusions 27 as shown in Fig. 6. The protrusions 27 are used to attach the nozzle 2 to the adapter 4.

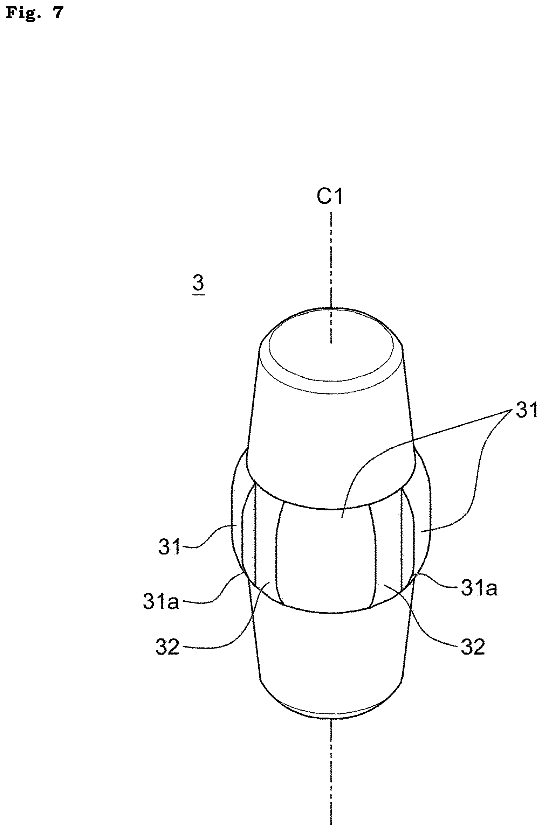

As shown in Fig. 2, the sub-nozzles 21 receive the substantially cylindrical rods 3 in their internal spaces. Fig. 7 is a perspective view of a rod 3 in Fig. 2. Fig. 7 shows an imaginary axis C1 for the rod 3 located in the internal space of the corresponding sub-nozzle 21. For ease of explanation, each rod 3 described below is in the internal space of the corresponding sub-nozzle 21 (refer to Fig. 5).

As shown in Fig. 7, the rod 3 includes raised portions 31 on its middle portion in the axial direction of the imaginary axis C1. The raised portions 31 protrude outward and extend along the imaginary axis C1. **In** the present embodiment, four raised portions 31 are at intervals of 90° in the circumferential direction of the imaginary axis C1. Fig. 7 shows three of the four raised portions 31. The number of raised portions 31 and the shape of the raised portions 31 are not limited to the above. For example, the rod 3 may include three or fewer or five or more raised portions 31. As described above, the rod 3 has a barrel shape with a raised middle portion. Each raised portion 31 has a slope 31a that is farther from the imaginary axis C1 toward the distal end 21a of the corresponding sub-nozzle 21 along the imaginary axis C1.

The rod 3 also has grooves 32 each between adjacent raised portions 31. The grooves 32 are recessed inward from the raised portions 31 and extend in a direction in which the rod 3 extends. The rod 3 thus has four grooves 32. Fig. 7 shows two of the four grooves 32.

The rod 3 may be symmetric in the direction in which the rod 3 extends. In other words, the rod 3 may have its upper half and the lower half symmetric to each other. The rod 3 can be placed in the internal space of the corresponding sub-nozzle 21 in the vertical orientation with either end placed upward. This facilitates the manufacture of the sprayer 1.

The rod 3 is formed from, for example, a synthetic resin material, such as polypropylene, high-density polyethylene, low-density polyethylene, linear low-density polyethylene, polyacetal, or polybutylene terephthalate, by injection molding.

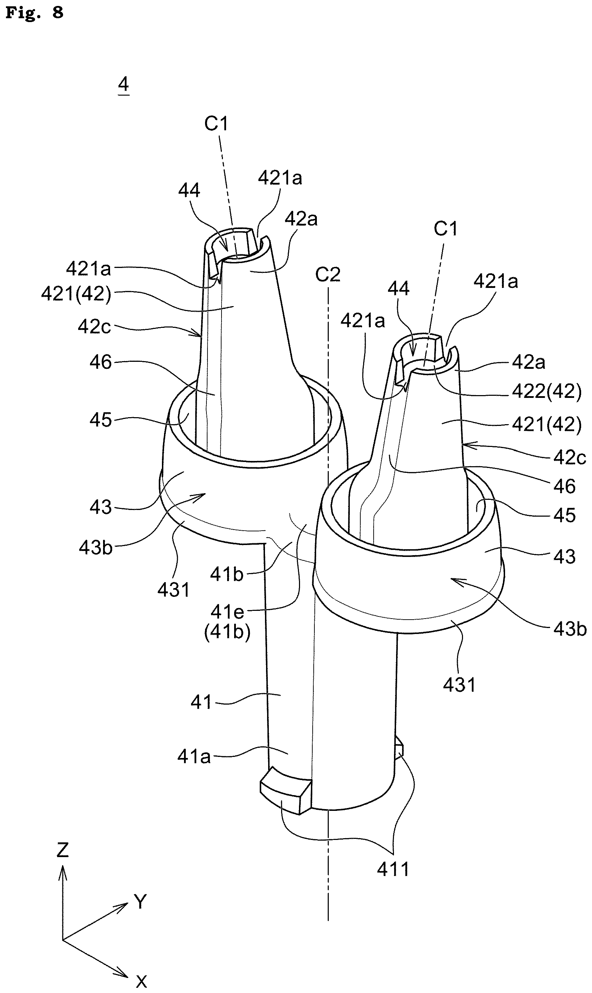

Fig. 8 is a perspective view of the adapter 4 in Fig. 2. As shown in Fig. 8, the adapter 4 includes the coupling pipe 41 connectable to the syringe 9, two cylindrical cores 42 connected to the coupling pipe 41, and circumferential walls 43 each surrounding the corresponding core 42. The adapter 4 is formed from, for example, a synthetic resin material, such as polypropylene, high-density polyethylene, low-density polyethylene, or linear low-density polyethylene, a styrene elastomer material, or an olefin elastomer material by injection molding.

Figs. 5 and 8 each show the single imaginary axis C2 extending parallel to Z-axis. As shown in Figs. 5 and 8, the coupling pipe 41 is cylindrical and centered on the imaginary axis C2. In other words, the coupling pipe 41 extends along the imaginary axis C2 and Z-axis. The imaginary axis C2 does not match the imaginary axes C1.

As shown in Fig. 8, the coupling pipe 41 has a coupling end 41a connectable to the syringe 9, and a connection end 41b opposite to the coupling end 41a. The coupling end 41a of the coupling pipe 41 is open. The connection end 41b of the coupling pipe 41 is closed by a closer 41e in the coupling pipe 41, except at holes 451 in receivers 45 (described later).

In the example shown in Fig. 5, the coupling pipe 41 is integral with the two cores 42. The coupling pipe 41 described herein has its upper end in the axial direction of the imaginary axis C2 being the connection between the closer 41e in the coupling pipe 41 and the circumferential walls 43. In other words, the coupling pipe 41 has a length from an opening in the coupling end 41a to the upper end in the axial direction of the imaginary axis C2. In Fig. 5 as well as Figs. 10 and 13 (referred to later), a two-dot-dash line L1 indicates the position of the upper end of the coupling pipe 41 in the axial direction of the imaginary axis C2.

As shown in Fig. 8, the coupling pipe 41 includes, on the coupling end 41a, two coupling projections 411 protruding away from the imaginary axis C2. The two coupling projections 411 are at intervals of 180° in the circumferential direction of the coupling pipe 41. The coupling projections 411 are used to connect the syringe 9 to the adapter 4. For example, each coupling projection 411 has a shape complying with a standard such as ISO 80369-7. The number of coupling projections 411 and the shape of the coupling projections 411 are not limited to the above when the coupling pipe 41 can be connected to a container such as the syringe 9 without liquid leakage. For example, the number of coupling projections 411 and the shape of the coupling projections 411 may be determined based on the container such as the syringe 9 to be connected to the adapter 4. The coupling projections 411 may not be located on the coupling pipe 41.

With the nozzle 2 attached to the adapter 4, the cores 42 are located in the internal spaces of the sub-nozzles 21 as shown in Fig. 5. In this state, each core 42 is coaxial with the corresponding imaginary axis C1. The two cores 42 thus extend along the respective imaginary axes C1 and are adjacent to each other along the imaginary axis C2.

As shown in Figs. 5 and 8, each core 42 has an upper distal end 42a and a lower basal end 42b. Each core 42 also has a cylindrical side surface 421 extending along the corresponding imaginary axis C1 and a top surface 422 at the distal end 42a. The top surface 422 is located downward from the edge of the distal end 42a of the core 42 in the axial direction of the imaginary axis C1. The distal end 42a of the core 42 thus includes a recessed portion 44 surrounded by the side surface 421 and the top surface 422.

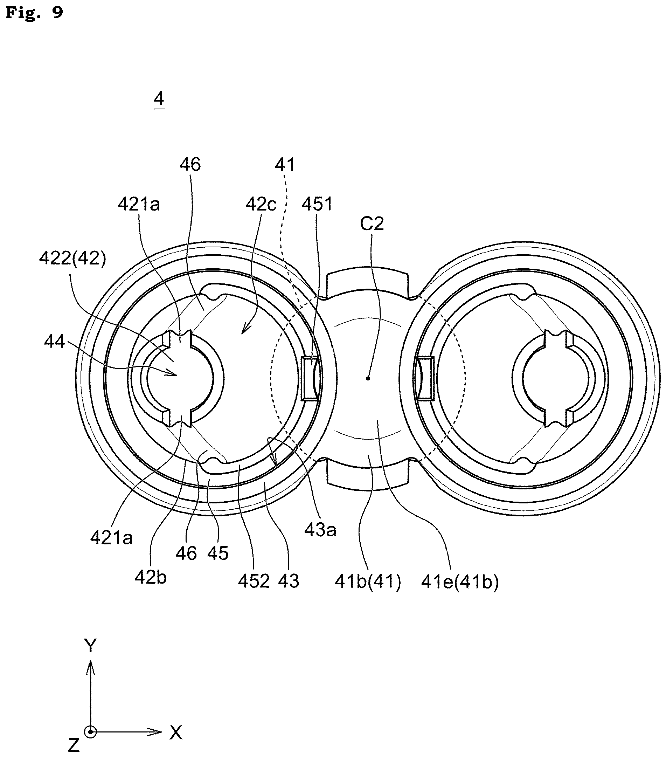

As shown in Fig. 8, the side surface 421 of each core 42 has two cutouts 421a. The cutouts 421a are open in the radial direction of the core 42 to the recessed portion 44. Fig. 9 is a plan view of the adapter 4 in Fig. 2. In Fig. 9, the components located leftward from the imaginary axis C2 alone are given reference numerals. As shown in Fig. 9, the two cutouts 421a are at, for example, intervals of 180° in the circumferential direction of the corresponding core 42.

As shown in Fig. 8, each side surface 421 has a radially outer side surface about the corresponding imaginary axis C1. The radially outer side surface is an outer side surface 42c of the corresponding core 42. The outer side surface 42c of each core 42 has grooves 46 extending between the basal end 42b of the core 42 and the cutouts 421a. In the example shown in Fig. 9, the outer side surface 42c has two grooves 46 at intervals of 180° in the circumferential direction of the core 42. The number of grooves 46 and the arrangement of the grooves 46 are not limited to this example. For example, one groove 46 or three or more grooves 46 may be arranged in the circumferential direction of the corresponding imaginary axis C1. For a structure with a core path 65 (described later), the grooves 46 may be eliminated.

As shown in Fig. 9, each core 42 has an edge defined by the basal end 42b of the core 42 in a plan view of the adapter 4 viewed from above along the imaginary axis C2. In other words, all the components of the core 42 are located inward from the basal end 42b of the core 42 in a plan view of the adapter 4.

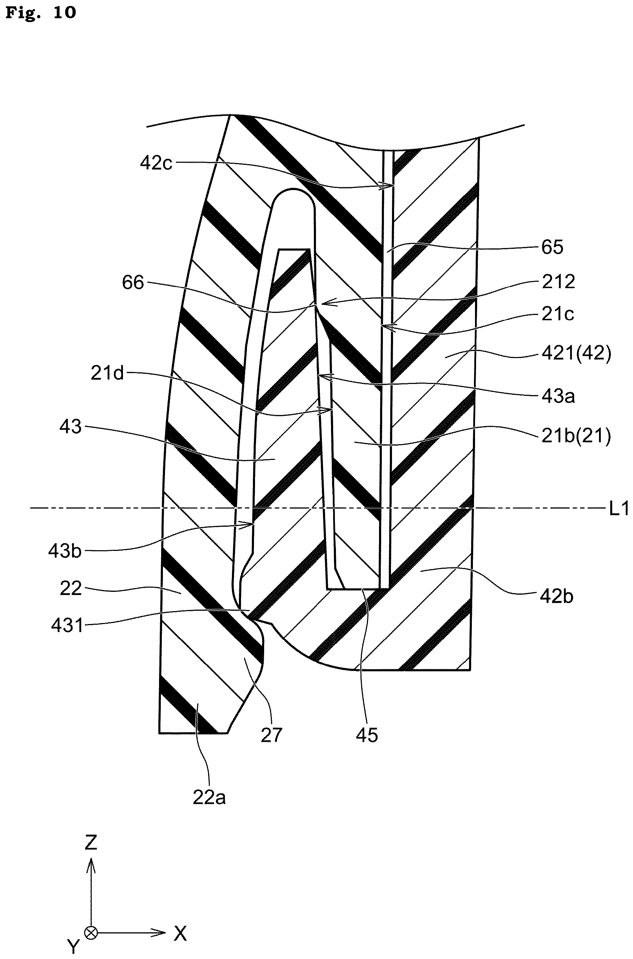

As shown in Figs. 5, 8, and 9, each circumferential wall 43 extends along the corresponding imaginary axis C1 and surrounds the basal end 42b of the corresponding core 42 in a plan view of the adapter 4 (refer to Fig. 9). Fig. 10 is a partially enlarged view of a receiver 45 in Fig. 5. As shown in Fig. 10, the circumferential wall 43 is connected to the basal end 42b of the corresponding core 42. This defines the annular, U-shaped receiver 45 that is open upward between the side surface 421 of the core 42 and the circumferential wall 43. Being U-shaped is not limited to being in a U-shape with a curved bottom portion, and includes being in a U-shape with a bottom portion having sharp corners as shown in Fig. 10. Each receiver 45 herein includes the side surface 421 of the corresponding core 42, the circumferential wall 43, and an annular, U-shaped space surrounded by the side surface 421 and the circumferential wall 43.

Each circumferential wall 43 has a radially inner side surface about the corresponding imaginary axis C1. The radially inner side surface is a facing surface 43a facing the outer side surface 42c of the corresponding core 42. The facing surface 43a of the circumferential wall 43 is an example of a facing surface of a receiver in an aspect of the present disclosure. With the nozzle 2 attached to the adapter 4, the circumferential wall 43 is inclined away from the outer side surface 42c of the core 42 toward the opening in the receiver 45 in the axial direction of the imaginary axis C1 as shown in Fig. 10. In other words, the receiver 45 has a groove with a width gradually increasing from its bottom toward its opening.

Each circumferential wall 43 has an outer surface 43b opposite to the facing surface 43a. As shown in Figs. 5 and 8, the outer surface 43b of each circumferential wall 43 includes an annular projection 431 protruding outward in the radial direction of the corresponding sub-nozzle 21. As shown in Fig. 10, the projection 431 is adjacent to the bottom of the receiver 45 in the axial direction of the imaginary axis C1. The projection 431 is used to connect the adapter 4 and the nozzle 2.

As shown in Fig. 9, the basal end 42b of each core 42 and the corresponding circumferential wall 43 are connected to the connection end 41b of the coupling pipe 41. In the present embodiment, the cores 42, the circumferential walls 43, and the receivers 45 are all symmetric about the imaginary axis C2.

The dashed line in Fig. 9 indicates the edge of the coupling pipe 41. The coupling pipe 41 overlaps a portion of each circumferential wall 43 in a plan view of the adapter 4. The coupling pipe 41 does not overlap the entire portion of each circumferential wall 43. In other words, each circumferential wall 43 includes a portion overlapping the coupling pipe 41 and a portion not overlapping the coupling pipe 41 in a plan view of the adapter 4. In the present embodiment, the coupling pipe 41 also overlaps a portion of each receiver 45 and a portion of each core 42.

A hole 451 is located in an overlapping portion of each receiver 45 and the coupling pipe 41. The hole 451 connects the receiver 45 and the coupling pipe 41 to allow fluid passage. In the present embodiment, each hole 451 extends through the bottom of the corresponding receiver 45 in Z-direction. Each hole 451 may extend through, for example, the corresponding circumferential wall 43 in X-direction.

Each receiver 45 has, on its bottom, a recessed groove 452 extending from the hole 451 to the end of each groove 46. The recessed groove 452 allows the liquid passing through the hole 451 to easily flow through the receiver 45 in the circumferential direction of the imaginary axis C1.

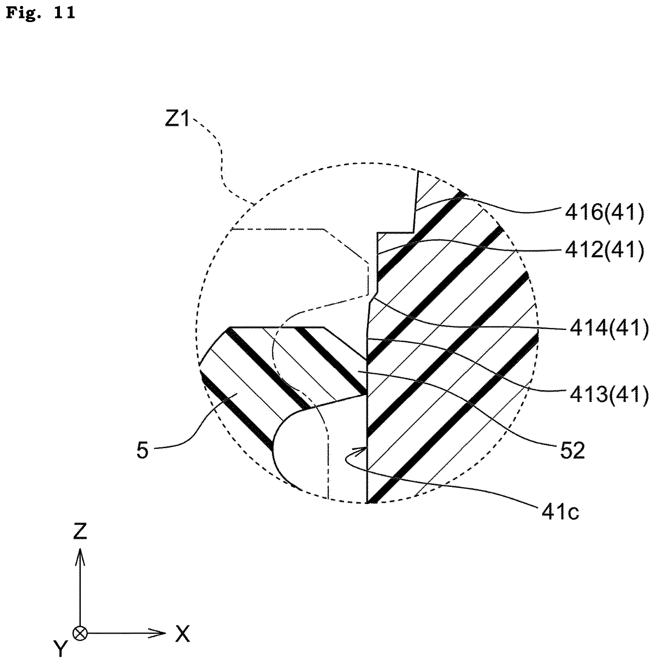

Fig. 11 is a partially enlarged view of area Z1 in Fig. 5. As shown in Fig. 11, the coupling pipe 41 includes a large cross-sectional portion (large-diameter portion) 412 nearer the coupling end 41a (downward in Fig. 11) than the two holes 451 and a small cross-sectional portion (small-diameter portion) 413 nearer the coupling end 41a than the large cross-sectional portion 412. The small cross-sectional portion 413 has a smaller inner diameter than the large cross-sectional portion 412. For example, the small cross-sectional portion 413 has a smaller area than the large cross-sectional portion 412 in the XY cross section. In other words, the area surrounded by the small cross-sectional portion 413 is smaller than the area surrounded by the large cross-sectional portion 412 on an XY plane when viewed in Z-direction.

The coupling pipe 41 includes an expanded portion 414 between the large cross-sectional portion 412 and the small cross-sectional portion 413. The expanded portion 414 has an inner diameter and an XY cross section gradually increasing toward the large cross-sectional portion 412 in Z-direction. In other words, the coupling pipe 41 has an axial inner surface 41c that is inclined away from the imaginary axis C2 toward the large cross-sectional portion 412 in the expanded portion 414.

The coupling pipe 41 has a recess 416 recessed outward above the large cross-sectional portion 412. The recess 416 is located below the holes 451 (refer to Fig. 5) in the receivers 45 in the axial direction of the imaginary axis C2. For example, the recess 416 extends from the holes 451 toward the coupling end 41a of the coupling pipe 41 along the imaginary axis C2.

As shown in Fig. 5, the coupling pipe 41 receives the substantially cylindrical stopper 5 extending along the imaginary axis C2 in its internal space. The stopper 5 is coaxial with the imaginary axis C2. The stopper 5 described below is located in the internal space of the coupling pipe 41.

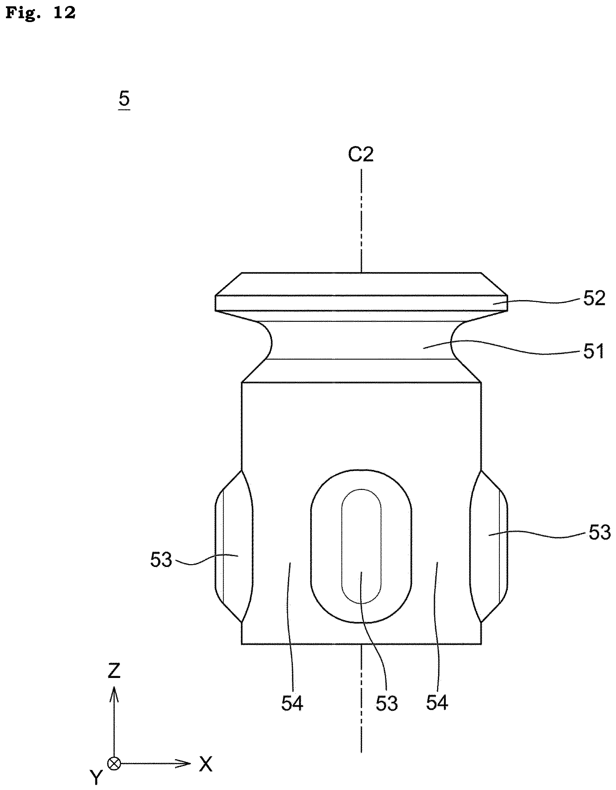

Fig. 12 is a side view of the stopper 5 in Fig. 2. FIG. 12 shows X-axis, Y-axis, and Z-axis as well as the imaginary axis C2 for the stopper 5 located in the internal space of the coupling pipe 41. As shown in Fig. 12, the stopper 5 includes a small-diameter portion 51 with a reduced diameter. The stopper 5 includes a liquid stopper 52 with a larger diameter than the small-diameter portion 51 nearer the connection end 41b of the coupling pipe 41 (downward in Fig. 12) than the small-diameter portion 51. As shown in Fig. 11, the liquid stopper 52 in the stopper 5 is in contact with the inner surface 41c of the coupling pipe 41 in a liquid tight manner along the entire circumference.

As shown in Fig. 12, the stopper 5 includes four protruding portions 53 protruding outward and extending along the imaginary axis C2 nearer the coupling end 41a of the coupling pipe 41 than the small-diameter portion 51. The four protruding portions 53 are at intervals of 90° in the circumferential direction of the stopper 5. Fig. 12 shows three of the four protruding portions 53. The protruding portions 53 are in contact with the inner surface 41c in the small cross-sectional portion 413 in the coupling pipe 41. The stopper 5 has four grooves 54 each between circumferentially adjacent protruding portions 53. The grooves 54 are recessed inward from the protruding portions 53 and extend along the imaginary axis C2. Fig. 12 shows two of the four grooves 54. As shown in Fig. 5, the stopper 5 has a recess 55 recessed toward the coupling end 41a along the imaginary axis C2.

The stopper 5 is formed from, for example, a synthetic resin material, such as polypropylene, high-density polyethylene, low-density polyethylene, or linear low-density polyethylene, a styrene elastomer material, an olefin elastomer material, or synthetic rubber. For example, the stopper 5 is formed by injection molding.

The syringe 9 connectable to the sprayer 1 may have a known structure. For example, as shown in FIG. 2, the syringe 9 includes an outer cylinder 91, a cylindrical connector 92 at a distal end 91a of the outer cylinder 91, and a plunger 93 insertable into the outer cylinder 91. The plunger 93 has a gasket 94 at its end that is insertable into the outer cylinder 91.

The connector 92 has, on its inner surface, a spiral groove (not shown) for receiving the sprayer 1 and an instrument such as a syringe needle by screwing. The groove has a shape complying with, for example, a standard such as ISO 80369-7. The gasket 94 slides inside the outer cylinder 91 together with the plunger 93 while being in contact with the inner surface of the outer cylinder 91 in a liquid tight manner. The plunger 93 is pushed toward the distal end 91a of the outer cylinder 91 to discharge the liquid retained inside the outer cylinder 91 from the distal end 91a.

### (2. Assembly of sprayer 1)

The assembly of the sprayer 1 will now be described. The rods 3 are located in the internal spaces of the sub-nozzles 21 with the raised portions 31 in contact with the projections 26 on the sub-nozzles 21. More specifically, the slopes 31a of the raised portions 31 are in contact with portions of the projections 26 nearer the distal ends 21a than the tops of the projections 26. This urges the rods 3 toward the distal ends 21a of the sub-nozzles 21 along the imaginary axes C1. The rods 3 are thus in contact with the distal ends 21a of the sub-nozzles 21 more reliably.

As shown in Fig. 5, a spray chamber 61 is defined between the recess 24 on each sub-nozzle 21 and the corresponding rod 3. The spray chamber 61 connects with the corresponding nozzle bore 23 to allow fluid passage. Guide paths 62 are defined between the grooves 25 on each sub-nozzle 21 and the corresponding rod 3. The guide paths 62 connect with the corresponding spray chamber 61 to allow fluid passage. Rod paths 63 are defined between the inner side surface 21c of each sub-nozzle 21 and the corresponding rod 3. The rod paths 63 connect with the corresponding guide paths 62 to allow fluid passage. The rod paths 63 may partly include the grooves 32 on the corresponding rod 3.

The adapter 4 is attached to the nozzle 2 with the cores 42 received in the respective sub-nozzles 21. As shown in Fig. 10, the basal end 21b of each sub-nozzle 21 is received in the corresponding receiver 45. In the present embodiment, the basal end 21b of each sub-nozzle 21 is in contact with the bottom of the corresponding receiver 45. Each sub-nozzle 21 has an outer surface 21d in contact with the facing surface 43a of the corresponding circumferential wall 43 in a liquid tight manner in the corresponding receiver 45. In other words, the outer surface 21d of each sub-nozzle 21 and the facing surface 43a of the corresponding circumferential wall 43 form a contact 66.

In the example shown in Fig. 10, each sub-nozzle 21 includes, on the outer surface 21d, an annular corner 212 extending along the entire circumference of the sub-nozzle 21 and facing the facing surface 43a of the corresponding circumferential wall 43. The corner 212 is located upward from the coupling pipe 41 in the axial direction of the imaginary axis C2 (in other words, in Z-direction). In Fig. 10, the two-dot-dash line L1 indicates the position of the upper end of the coupling pipe 41 in the axial direction of the imaginary axis C2.

Each contact 66 is thus located upward from the coupling pipe 41 in the axial direction of the imaginary axis C2 and is linear along the entire circumference of the corresponding receiver 45. Although the contact 66 may be planar, the contact 66 may be linear as in the present embodiment. The linear contact 66 allows the force from the circumferential wall 43 and the sub-nozzle 21 pushing each other to be concentrated in a smaller area than a planar contact 66. This increases contact pressure between the circumferential wall 43 and the sub-nozzle 21 at the contact 66.

In the present embodiment, the basal end 21b of each sub-nozzle 21 and the corresponding circumferential wall 43 are in contact with each other at the corresponding contact 66 and thus inclined away from each other in the radial direction of the corresponding imaginary axis C1. In other words, the basal end 21b of each sub-nozzle 21 is inclined toward the corresponding imaginary axis C1 in the negative direction along Z-axis. Each circumferential wall 43 is inclined away from the corresponding imaginary axis C1 in the positive direction along Z-axis. Either of the basal end 21b of the sub-nozzle 21 and the circumferential wall 43 in contact with each other may be inclined, or both may not be inclined.

The projection 431 on each circumferential wall 43 is nearer the corresponding nozzle bore 23 than the corresponding lower edge 22a of the nozzle 2 in the axial direction of the imaginary axis C1 and is in contact with the corresponding protrusion 27 on the nozzle 2. This reduces the likelihood that the nozzle 2 is detached from the adapter 4 unintentionally. The adapter 4 thus remains attached to the nozzle 2.

Each recessed portion 44 in the adapter 4 receives a part of the corresponding rod 3. A chamber 64 is defined between the distal end 42a of each core 42 and the corresponding rod 3. The chamber 64 connects with the corresponding rod paths 63. The chamber 64 may partly include the recessed portion 44 when the rod 3 is not in contact with the bottom of the recessed portion 44, or in other words, the top surface 422 of the core 42 (refer to Fig. 5).

A core path 65 is defined between the inner side surface 21c of each sub-nozzle 21 and the outer side surface 42c of the corresponding core 42. The core path 65 connects the corresponding chamber 64 and the corresponding hole 451 to allow fluid passage. The core path 65 is divided from outside the core path 65 by the corresponding contact 66 in a liquid tight manner.

The sprayer 1 with the above structure includes flow channels each including the hole 451, the core path 65, the chamber 64, the rod paths 63, the guide paths 62, the spray chamber 61, and the nozzle bore 23.

### (3. Spraying Operation)

The operation for spraying the liquid from the sprayer 1 will now be described. First, the syringe 9 is filled with liquid to be sprayed. For example, for the syringe 9 with the connector 92 having a shape complying with ISO 80369-7, an instrument such as a syringe needle and a tube complying with the same standard is connectable to the connector 92. The liquid can be sucked from a liquid container such as a vial and an ampule through, for example, a syringe needle connected to the connector 92 to fill the syringe 9. The syringe 9 may be a prefilled syringe containing prefilled liquid.

The coupling projections 411 on the coupling pipe 41 are then screwed into the connector 92 in the syringe 9 to connect the sprayer 1 and the syringe 9 (refer to Fig. 1). The plunger 93 in the syringe 9 is then pushed toward the distal end 91a of the outer cylinder 91. The plunger 93 described below is continuously pushed until spraying is complete.

As the plunger 93 is pushed, the liquid in the syringe 9 enters the coupling pipe 41. In this state, the liquid stopper 52 in the stopper 5 is in contact with the inner surface 41c in a liquid tight manner in the small cross-sectional portion 413 (refer to Fig. 11) in the coupling pipe 41. The liquid thus cannot flow toward the connection end 41b beyond the liquid stopper 52. Thus, the air in the coupling pipe 41 is compressed between the gasket 94 and the liquid stopper 52 in the stopper 5.

When the plunger 93 is pushed harder, the stopper 5 is pressed under a force exceeding the maximum static friction force against the inner surface 41c of the coupling pipe 41 and starts sliding toward the connection end 41b of the coupling pipe 41. The magnitude of the force to be applied to the liquid to cause the stopper 5 to start sliding is equal to or greater than the magnitude of the force to be applied to the liquid for spraying.

Once the stopper 5 starts sliding, the stopper 5 is pressed by the plunger 93 through the liquid and is also pressed under the repulsive force of the compressed air. The stopper 5 thus accelerates suddenly. When the liquid stopper 52 in the stopper 5 reaches the expanded portion 414 in the coupling pipe 41, the stopper 5 is pressed further toward the connection end 41b of the coupling pipe 41 by the contact between the liquid stopper 52 and the inclined inner surface 41c of the coupling pipe 41. Thus, the stopper 5 accelerates further.

As indicated by the two-dot-dash line in Fig. 11, when reaching the large cross-sectional portion 412 in the coupling pipe 41, the liquid stopper 52 in the stopper 5 separates from the inner surface 41c of the coupling pipe 41. This allows the compressed liquid to flow toward, in the coupling pipe 41, the connection end 41b (upward in Fig. 11) beyond the liquid stopper 52 in the stopper 5.

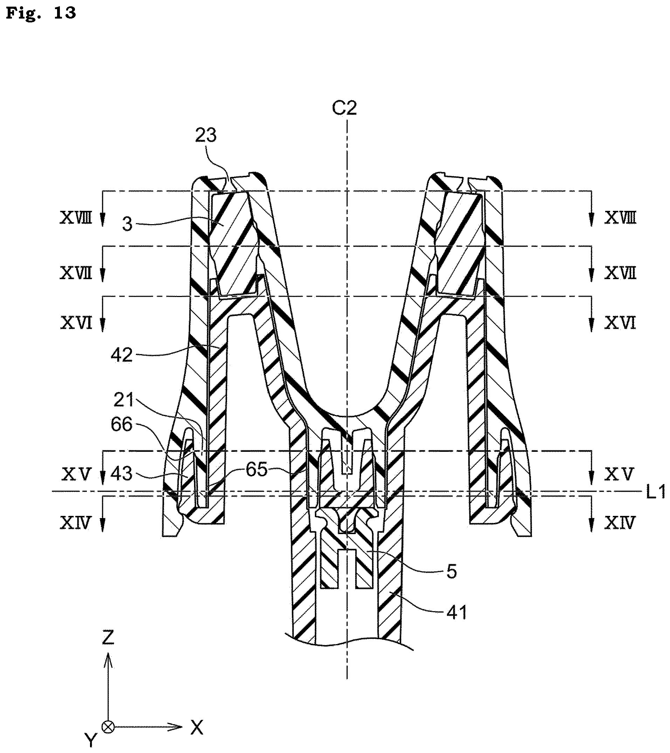

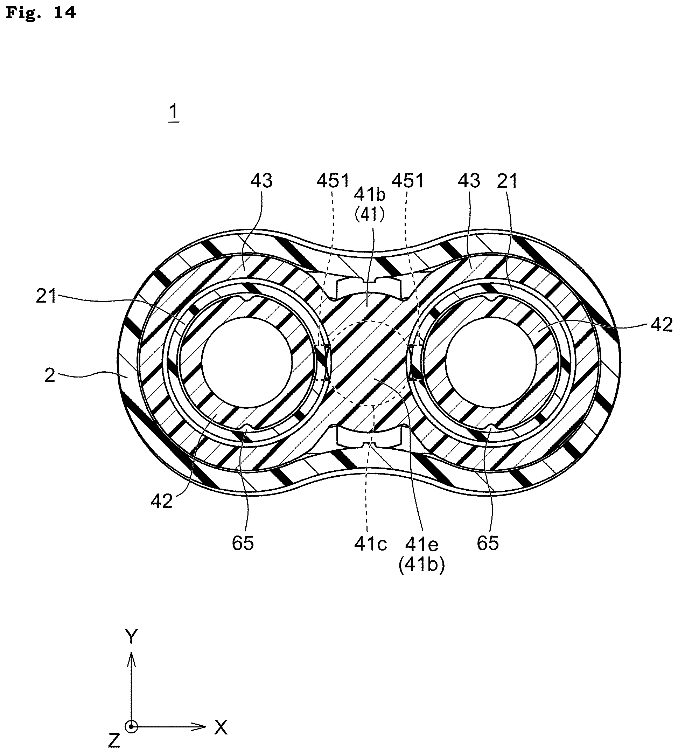

Fig. 13 is an end view of the sprayer 1 in Fig. 4 taken along line V-V. In Fig. 13, the components located leftward from the imaginary axis C2 alone are given reference numerals. Unlike Fig. 5, Fig. 13 shows the stopper 5 located in the large cross-sectional portion 412 in the coupling pipe 41. Fig. 14 is a cross-sectional view of the sprayer 1 in Fig. 13 taken along line XIV-XIV. The circular dashed line in Fig. 14 indicates the inner surface 41c at the connection end 41b of the coupling pipe 41. As shown in Fig. 14, the liquid passes through the holes 451 in the axial direction of the imaginary axis C2 and enters the core paths 65 and the clearances between the outer surfaces 21d of the sub-nozzles 21 and the facing surfaces 43a of the circumferential walls 43. The liquid fills the core path 65 in the circumferential direction of the sub-nozzles 21 and flows through the core paths 65 in the axial direction of the imaginary axes C1 toward the nozzle bore 23.

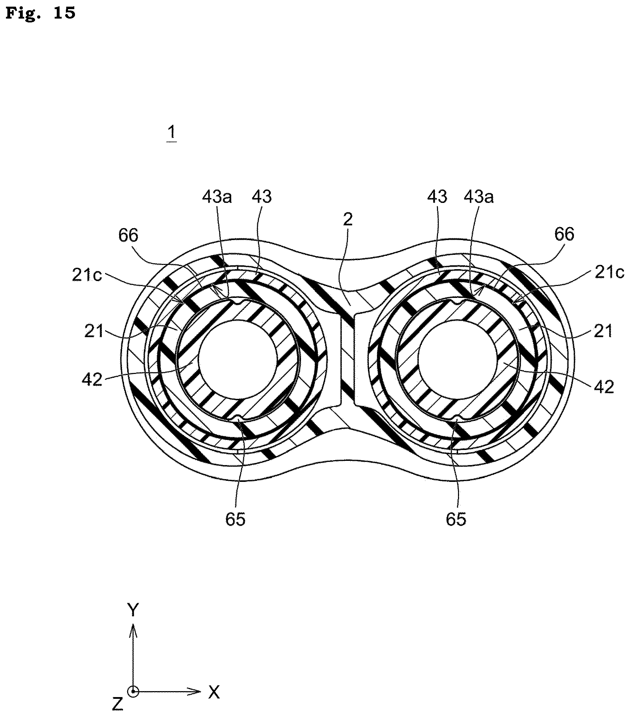

Fig. 15 is a cross-sectional view of the sprayer 1 in Fig. 13 taken along line XV-XV. More specifically, Fig. 15 is a cross-sectional view of the sprayer 1 taken along a plane near the openings in the receivers 45 (refer to Fig. 13) in the axial direction of the imaginary axis C2. As shown in Fig. 15, the outer surface 21d of each sub-nozzle 21 and the facing surface 43a of the corresponding circumferential wall 43 are in contact with each other in a liquid tight manner along the entire circumference. **In** other words, the outer surface 21d of each sub-nozzle 21 and the facing surface 43a of the corresponding circumferential wall 43 form the contact 66.

Each contact 66 is in the shape of a perfect circle or substantially a perfect circle in a cross section intersecting with the imaginary axis C2. The roundness of each contact 66 in the above cross section may be less than or equal to 0.1 times, more specifically, less than or equal to 0.05 times, or more still specifically, less than or equal to 0.025 times. Roundness is the measure that indicates the degree of deviation of a circle to be measured from a geometrically perfect circle. For example, the roundness of a circle is measured as the difference between the radii of two geometrically perfect circles, concentric with the circle to be measured, that are placed to sandwich the circle and moved to minimize the distance between the two circles. Roundness herein is the ratio of the above difference between the radii to the radius of the smaller one of the two circles. For example, when the smaller one of the two circles has a radius of 4 mm, the difference may be less than or equal to 0.4 mm, more specifically less than or equal to 0.2 mm, or still more specifically, less than or equal to 0.1 mm.

The flattening of each contact 66 in the above cross section may be less than or equal to 1/6, more specifically, less than or equal to 1/12, or still more specifically, less than or equal to 1/24. Flattening is expressed as (a - b)/a, where a is the major radius and b is the minor radius of an ellipse.

For the nozzle 2 detached from the adapter 4, the outer surfaces 21d of the sub-nozzles 21 are in the shape of perfect circles or substantially perfect circles in a cross section intersecting with the imaginary axis C2 and including portions corresponding to the contacts 66 in the sub-nozzles 21. The roundness of the outer surface 21d of each sub-nozzle 21 in the above cross section may be less than or equal to 0.1 times, more specifically, less than or equal to 0.05 times, or still more specifically, less than or equal to 0.025 times. The flattening of the outer surface 21d of each sub-nozzle 21 in the above cross section may be less than or equal to 1/6, more specifically, less than or equal to 1/12, or still more specifically, less than or equal to 1/24. Such outer surfaces 21d of the sub-nozzles 21 can form the contacts 66 that are in the shape of perfect circles or substantially perfect circles in the cross section intersecting with the imaginary axis C2.

For the adapter 4 not receiving the nozzle 2, the facing surfaces 43a of the circumferential walls 43 are in the shape of perfect circles or substantially perfect circles in a cross section intersecting with the imaginary axis C2 and including portions in the circumferential walls 43 corresponding to the contacts 66. The roundness of the facing surface 43a of each circumferential wall 43 in the above cross section may be less than or equal to 0.1 times, more specifically, less than or equal to 0.05 times, or still more specifically, less than or equal to 0.025 times. The flattening of the facing surface 43a of each circumferential wall 43 in the above cross section may be less than or equal to 1/6, more specifically, less than or equal to 1/12, or still more specifically, less than or equal to 1/24. Such facing surfaces 43a of the circumferential walls 43 described above can form the contacts 66 that are in the shape of perfect circles or substantially perfect circles in the cross section intersecting with the imaginary axis C2.

In the present embodiment, each contact 66 is in the shape of a perfect circle in the cross section orthogonal to the imaginary axis C2, or in other words, the XY cross section. As shown in Fig. 10, each contact 66 divides the clearance between the corresponding sub-nozzle 21 and the corresponding circumferential wall 43, or in other words, the space connecting with the core path 65 for fluid passage from outside the clearance or the space in a liquid tight manner.

Each core path 65 includes a portion with its channel cross section smaller than the channel cross section of the holes 451. This increases the flow velocity of the liquid in this portion of the core path 65, and thus shortens the time taken for the liquid to pass through the flow channel from the hole 451 to the nozzle bore 23. In other words, this shortens the time from when the liquid stopper 52 in the stopper 5 separates from the inner surface 41c of the coupling pipe 41 to when the liquid starts being sprayed from the nozzle bores 23. This improves the operability during spraying.

A cross section or an open face larger or smaller than another cross section or another open face herein refers to the cross section or the open face having at least one of an area, a diameter, or a circumference larger or smaller than the corresponding area, diameter, or circumference of the other cross section or the other open face. When the size of a cross section or an open face of, for example, a path in each flow channel is compared and when each flow channel includes multiple paths, the total size of the cross sections or the open faces of the multiple paths are used herein for comparison.

A channel cross section herein refers to, of the cross sections of the channel, a cross section orthogonal to a direction in which the liquid flows. **In** the present embodiment, the liquid flows through the holes 451 along the imaginary axis C2. The channel cross section of each hole 451 is thus a cross section intersecting with the imaginary axis C2.

As shown in Fig. 13, the core paths 65 extend along the imaginary axis C2. In other words, the liquid flows through the core paths 65 along the imaginary axis C2. The channel cross section of each core path 65 is thus a cross section intersecting with the imaginary axis C2.

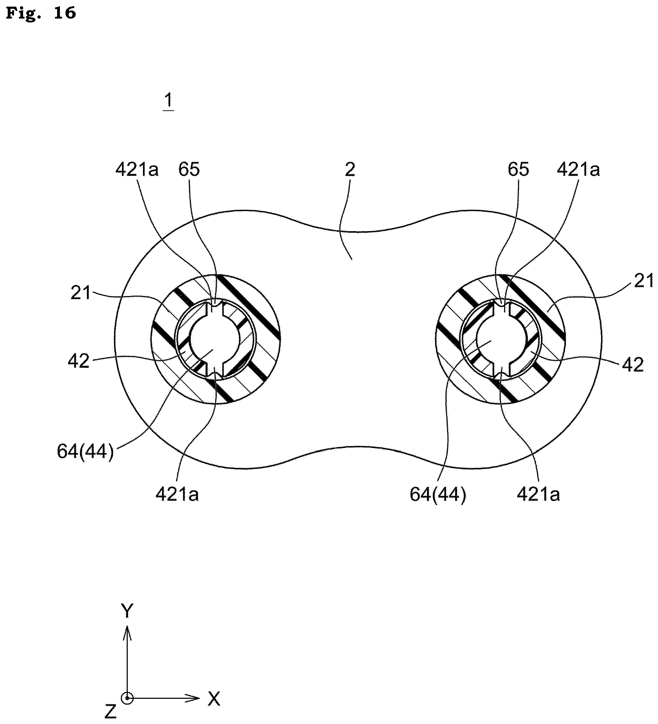

Fig. 16 is a cross-sectional view of the sprayer 1 in Fig. 13 taken along line XVI-XVI. As shown in Fig. 16, the liquid passing through the core paths 65 enters the recessed portions 44, or in other words, the chambers 64, through the cutouts 421a.

Each chamber 64 includes a portion with its channel cross section larger than the channel cross section of the core paths 65. In the present embodiment, the liquid flows through the chambers 64 along the imaginary axes C1. The channel cross section of each chamber 64 is thus a cross section intersecting with the corresponding imaginary axis C1. The sub-nozzles 21 extend along the imaginary axis C2. The channel cross section of each chamber 64 is thus a cross section intersecting with the imaginary axis C2.

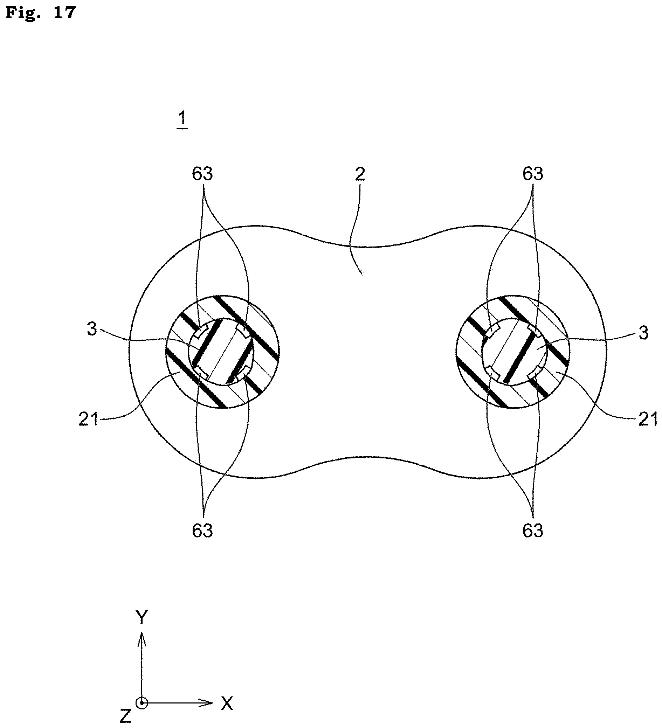

Fig. 17 is a cross-sectional view of the sprayer 1 in Fig. 13 taken along line XVII-XVII. As shown in Fig. 17, the liquid passing through the chambers 64 enters the rod paths 63. Each rod path 63 includes a portion with its channel cross section smaller than the channel cross section of the chambers 64. In the present embodiment, the rod paths 63 extend along the corresponding imaginary axes C1 and the imaginary axis C2. In other words, the liquid flows through the rod paths 63 along the imaginary axes C1 and the imaginary axis C2. The channel cross section of each rod path 63 is thus a cross section intersecting with the corresponding imaginary axis C1 or the imaginary axis C2.

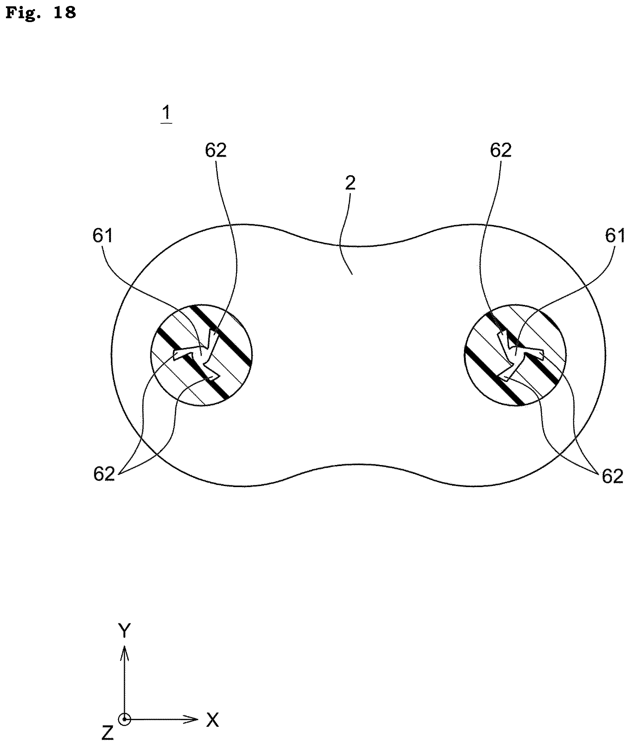

Fig. 18 is a cross-sectional view of the sprayer 1 in Fig. 13 taken along line XVIII-XVIII. As shown in Fig. 18, the liquid passing through the rod paths 63 enters the guide paths 62. Each guide path 62 includes a portion with its channel cross section smaller than the channel cross section of the rod paths 63. This accelerates the liquid flowing into the guide paths 62 from the rod paths 63. This structure thus achieves a sufficient flow velocity for spraying in the nozzle bores 23 more easily than a structure in which the guide paths 62 have a larger channel cross section than the rod paths 63. In the present embodiment, the guide paths 62 extend radially from the corresponding imaginary axes C1. The liquid thus flows through the guide paths 62 radially from the corresponding imaginary axes C1. The channel cross section of each chamber 64 is thus a cross section in the radial direction of the corresponding imaginary axis C1.

The liquid passing through the guide paths 62 enters the spray chambers 61. Each guide path 62 is open to the corresponding spray chamber 61 in the circumferential direction of the spray chamber 61. The liquid thus flows spirally and is atomized in the spray chambers 61. The atomized liquid is sprayed through the nozzle bores 23 (refer to Fig. 4) into the nostrils in which the sub-nozzles 21 are placed.

Each nozzle bore 23 has an open face smaller than the channel cross section of the corresponding flow channel. In the present embodiment, each nozzle bore 23 has an open face smaller than the channel cross section of each of the guide paths 62, the rod paths 63, the chambers 64, the core paths 65, and the holes 451.

When a flow channel has a reduced channel cross section at a point, the flow velocity increases at the point, whereas the pressure to cause the liquid to pass through the flow channel increases. The nozzle bores 23 having the open faces smaller than the channel cross sections of the other portions in the flow channels can reduce the pressure to cause the liquid to pass through the flow channels while maintaining a sufficient flow velocity for spraying in the nozzle bores 23. The sprayer 1 thus has improved operability during spraying.

Although the container connected to the sprayer 1 is the syringe 9 in the above example, the container is not limited to this example. For example, a pump container (e.g., a nasal pump) that delivers compressed liquid may be connected to the sprayer 1. In this case, the liquid can be compressed in the container. Thus, the stopper 5 may be eliminated.

### (4. Advantageous Effects)

As described above, the sprayer 1 according to the present embodiment includes the nozzle 2 insertable into a nostril and the adapter 4 to connect the nozzle 2 and the syringe 9 containing liquid. The nozzle 2 includes at least one sub-nozzle 21. The sub-nozzle 21 has the distal end 21a and the basal end 21b. The distal end 21a has the nozzle bore 23 and is insertable into the nostril. The adapter 4 includes the coupling pipe 41, the two cores 42, and the annular, U-shaped receivers 45. The coupling pipe 41 has the coupling end 41a connectable to the syringe 9 to allow fluid passage. The two cores 42 each extend in the axial direction in which the coupling pipe 41 extends and have an axis different from the axis of the coupling pipe 41 in the radial direction intersecting with the axial direction. The two cores 42 are adjacent to each other. The receivers 45 are each at the outer side surface 42c and the basal end 42b of the corresponding core 42. The receivers 45 are each open toward the distal end 42a of the corresponding core 42. Each receiver 45 has the hole 451 connecting with the coupling pipe 41 to allow fluid passage. The nozzle 2 is attached to the adapter 4 with at least one of the two cores 42 received in the internal space of the sub-nozzle 21 and the basal end 21b of the sub-nozzle 21 received in the corresponding receiver 45. The sub-nozzle 21 and the core 42 define the core paths 65 between them. The core paths 65 connect with the coupling pipe 41 through the corresponding hole 451 to allow fluid passage. The core paths 65 are at least parts of the flow channel extending from the corresponding hole 451 to the corresponding nozzle bore 23. In an attachment state of the nozzle 2 attached to the adapter 4, the outer surface 21d of the sub-nozzle 21 and the facing surface 43a of the corresponding circumferential wall 43 facing the outer surface 21d are in contact with each other in a liquid tight manner and divide the core paths 65 from outside the core paths 65. In the attachment state, the contact 66 between the outer surface 21d of the sub-nozzle 21 and the facing surface 43a of the corresponding circumferential wall 43 is in the shape of a perfect circle or substantially a perfect circle in a cross section intersecting with the axial direction.

In this structure, the contact 66 between the outer surface 21d of the sub-nozzle 21 and the facing surface 43a of the corresponding circumferential wall 43 is in the shape of a perfect circle or substantially a perfect circle in a cross section intersecting with the axial direction. This distributes the pressure of the liquid acting to separate the contact 66 along the entire circumference of the contact 66. In other words, this reduces the likelihood that the pressure of the liquid is concentrated in a part of the contact 66. This reduces the likelihood of the contact 66 to be separated under concentrated pressure. The sprayer 1 is thus less likely to have liquid leakage.

To close a clearance between two components in a liquid tight manner, a sealing member such as a gasket or an O-ring may be placed between the two components. In such a structure, two contacts are formed between the two components and the sealing member. In contrast, in the structure described above, the sub-nozzle 21 and the corresponding circumferential wall 43 are in contact with each other without any sealing member. In other words, the sub-nozzle 21 and the corresponding circumferential wall 43 form one contact 66. This structure includes fewer contacts, through which liquid may leak, than a structure in which a sealing member is placed between the sub-nozzle 21 and the corresponding circumferential wall 43. The sprayer 1 is thus less likely to have liquid leakage.

In the sprayer 1 according to the present embodiment, the contact 66 is between the coupling pipe 41 and the nozzle bore 23 in the axial direction. In other words, the contact 66 is located upward from the coupling pipe 41 in Z-direction.

Each circumferential wall 43 has, in Z-direction, a portion adjacent to the coupling pipe 41 that is integral with the closer 41e in the coupling pipe 41 as shown in Fig. 14. In other words, the circumferential wall 43 has, in the portion described above, a thickness that is not uniform or not substantially uniform in a cross section intersecting with the axial direction. A plastic cylinder with varying thicknesses is likely to have a lower dimensional accuracy due to unevenly cured plastic. In other words, when the adapter 4 is formed from plastic, the roundness of the facing surface 43a of each circumferential wall 43 is likely to decrease in a cross section intersecting with the axial direction.

Each circumferential wall 43 has, in Z-direction, a portion upward from the coupling pipe 41 that is not integral with the coupling pipe 41 as shown in Fig. 15. In this portion, the circumferential wall 43 has a uniform or substantially uniform thickness. This causes less curing unevenness in the plastic. The roundness of the facing surface 43a of the circumferential wall 43 is thus less likely to decrease in a cross section intersecting with the axial direction. This allows more reliable formation of the contact 66 that is in the shape of a perfect circle or substantially a perfect circle in a cross section intersecting with the axial direction. The sprayer 1 is thus still less likely to have liquid leakage.

In the sprayer 1 according to the present embodiment, the facing surface 43a of each circumferential wall 43 is inclined away from the outer side surface 42c of the corresponding core 42 toward the opening in the corresponding receiver 45 in the axial direction of the corresponding imaginary axis C1 in the attachment state. The facing surface 43a of the circumferential wall 43 and the outer surface 21d of the sub-nozzle 21 are linearly in contact with each other at the contact 66.

This structure causes the force from the circumferential wall 43 and the sub-nozzle 21 pushing each other to be concentrated on the linear contact 66 more than a structure in which the facing surface 43a of the circumferential wall 43 and the outer surface 21d of the sub-nozzle 21 are planarly in contact with each other. This increases contact pressure between the circumferential wall 43 and the sub-nozzle 21 at the contact 66. The facing surface 43a of the circumferential wall 43 is inclined as described above. The circumferential wall 43 thus pushes the basal end 21b of the sub-nozzle 21 toward the core 42 through the contact 66. This reduces the eccentricity between the sub-nozzle 21 and the circumferential wall 43. This reduces the likelihood that liquid leaks through the contact 66. The sprayer 1 can thus further reduce liquid leakage.

In the sprayer 1 according to the present embodiment, each circumferential wall 43 includes the annular projection 431 protruding outward from the outer surface 43b being the surface opposite to the facing surface 43a. The nozzle 2 includes the protrusions 27 protruding toward the facing surface 43a of each circumferential wall 43 in the attachment state. In the attachment state, the protrusions 27 on the nozzle 2 are located farther from the nozzle bore 23 than the projections 431 on the circumferential walls 43 in the axial direction of the corresponding imaginary axes C1 and are in contact with the projection 431 on the corresponding circumferential wall 43.

In this structure, the protrusions 27 on the nozzle 2 are in contact with the projection 431 on the corresponding circumferential wall 43 from below in the axial direction of the corresponding imaginary axis C1. This allows the projection 431 on the circumferential wall 43 to function as a retainer for the nozzle 2, thus reducing the likelihood that the nozzle 2 is detached from the adapter 4 unintentionally.

As described above, the sub-nozzle 21 more easily moves in a direction in which the sub-nozzle 21 slips from the corresponding receiver 45 when the facing surfaces 43a of the circumferential walls 43 are inclined with respect to the outer side surfaces 42c of the respective cores 42 than when the facing surfaces 43a of the circumferential walls 43 are not inclined. In other words, the nozzle 2 is more likely to be detached from the adapter 4. In contrast, the above structure reduces the likelihood that the nozzle 2 is detached from the adapter 4 unintentionally. The facing surfaces 43a of the circumferential walls 43 may thus be inclined.

In the sprayer 1 according to the present embodiment, the nozzle 2 includes the two sub-nozzles 21. Each core 42 is received in the internal space of the corresponding sub-nozzle 21 in the attachment state. In the attachment state, each of the two contacts 66 is in the shape of a perfect circle or substantially a perfect circle in a cross section intersecting with the axial direction.

This structure reduces the likelihood of the two contacts 66 each being separated. The sprayer 1 is thus still less likely to have liquid leakage.

In the sprayer 1 according to the present embodiment, each core 42 has an edge defined by the basal end 42b of the core 42 in a plan view of the adapter 4.

In this structure, when the nozzle 2 including the two sub-nozzles 21 adjacent to and integral with each other is attached to the adapter 4, the two sub-nozzles 21 can be placed over the respective two cores 42 in the axial direction of the imaginary axis C2. In other words, the nozzle 2 is not to be deformed (e.g., bent) temporarily to place the sub-nozzles 21 over the cores 42. The nozzle 2 can thus be less flexible. The nozzle 2 being less flexible reduces deformation of the sub-nozzles 21 caused under the liquid pressure, thus reducing the likelihood that the liquid leaks through the contacts 66. The sprayer 1 is thus still less likely to have liquid leakage.

When the protrusions 27 on the nozzle 2 and the projections 431 on the circumferential walls 43 function as retainers, the nozzle 2 can also be less flexible when the protrusions 27 can move over the projections 431 downward in Z-direction during the attachment of the nozzle 2. The effects described above can thus be produced.

In the sprayer 1 according to the present embodiment, each sub-nozzle 21 has, in its internal space and between the distal end 21a of the sub-nozzle 21 and the distal end 42a of the corresponding core 42, the chamber 64 being at least a part of the flow channel. In the flow channel, the chamber 64 has a larger channel cross section than the core paths 65.

in this structure, the chamber 64 has a larger channel cross sections than the core paths 65, allowing the liquid flowing into the chamber 64 from the core paths 65 to decelerate. This reduces any difference between the leading ends of the flows of the liquid in the two flow channels. This reduces the difference in the start time for spraying at the two nozzle bores 23, thus reducing unevenness in the amount of liquid sprayed from the two nozzle bores 23. The liquid can thus be sprayed into both nostrils evenly.

The sprayer 1 according to the present embodiment further includes the rods 3 each in the internal space of the corresponding sub-nozzle 21 of the two sub-nozzles 21. Each rod 3 is between the distal end 21a of the corresponding sub-nozzle 21 and the distal end 42a of the corresponding core 42. The chamber 64 is between each rod 3 and the distal end 21a of the corresponding sub-nozzle 21. Each rod 3 and the inner side surface 21c of the corresponding sub-nozzle 21 define a rod path 63 between them.

This structure defines the chamber 64 between the distal end 21a of each sub-nozzle 21 and the distal end 42a of the corresponding core 42. This structure also defines the rod paths 63. The liquid can thus be guided from the core paths 65 toward the nozzle bores 23. This structure can supply the liquid to the nozzle bores 23 more stably than a structure including no rod 3, thus improving the stability in spraying the liquid.

In the sprayer 1 according to the present embodiment, the rod paths 63 have smaller channel cross sections than the chamber 64 in the flow channel.

In this structure, the rod paths 63, which are nearer the nozzle bores 23 than the chambers 64 in the flow channels, have smaller channel cross sections than the chambers 64. This can accelerate the liquid flowing into the rod paths 63 from the chambers 64. This structure can thus achieve a sufficient flow velocity for spraying in the nozzle bores 23 more easily than a structure in which the rod paths 63 have larger channel cross sections than the chambers 64.

In the sprayer 1 according to the present embodiment, each rod 3 includes the raised portions 31 protruding outward. Each sub-nozzle 21 includes the projections 26 protruding inward from the corresponding inner side surface 21c. In the attachment state, the projections 26 on each sub-nozzle 21 are located farther from the corresponding nozzle bore 23 than the raised portions 31 in the corresponding rod 3 in the axial direction of the corresponding imaginary axis C1 and are in contact with the raised portions 31 in the corresponding rod 3. Each rod 3 is in contact with the distal end 21a of the corresponding sub-nozzle 21. Each rod 3 and the distal end 21a of the corresponding sub-nozzle 21 define, between them, the guide paths 62 extending in a direction intersecting with the axial direction and connecting the corresponding rod paths 63 and the corresponding nozzle bore 23 to allow fluid passage.

For example, when the guide paths 62 are defined between the distal end 21a of the corresponding sub-nozzle 21 and the corresponding core 42 extending adjacent to the distal end 21a with no rod 3, manufacturing tolerances of the adapter 4 may create an unintended gap between the distal end 21a of the sub-nozzle 21 and the corresponding core 42. Such a gap increases the area of the channel cross sections of the guide paths 62, thus reducing the flow velocity of the liquid in the guide paths 62. In contrast, in the above structure, each rod 3 is in contact with the projections 26 on the corresponding sub-nozzle 21 and also in contact with the distal end 21a of the corresponding sub-nozzle 21 to define the guide paths 62. This reduces the likelihood that an unintended gap is created between the rod 3 and the distal end 21a of the corresponding sub-nozzle 21, thus maintaining the dimensional stability of the guide paths 62. This structure more reliably allows the liquid to flow at a flow velocity sufficient for spraying in the nozzle bores 23.

In the sprayer 1 according to the present embodiment, each core path 65 has a smaller channel cross section than the corresponding hole 451 in the flow channel.

In this structure, the core paths 65 have smaller channel cross sections than the holes 451. This allows the liquid to flow at a faster flow velocity in the core paths 65 than a structure in which the core paths 65 have larger channel cross sections than the holes 451. This shortens the time taken for the leading end of the flow of liquid to pass through each flow channel, thus reducing the time lag between the start of applying pressure on the liquid and the start of spraying. The sprayer 1 thus has improved operability during spraying.

In the sprayer 1 according to the present embodiment, each receiver 45 includes a portion overlapping the coupling pipe 41 and a portion not overlapping the coupling pipe 41 in a plan view of the adapter 4.

To reduce leakage of the liquid from the connection between the coupling pipe 41 and the syringe 9, the coupling end 41a of the coupling pipe 41 may have a cross section that is in the shape of a perfect circle or substantially a perfect circle. To obtain such a cross section using plastic, the coupling pipe 41 may be a cylinder having a cross section that is in the shape of a perfect circle or substantially a perfect circle along its entire length. When the coupling pipe 41 with this structure overlaps the entire portions of the two receivers 45, the coupling pipe 41 increases in size in Y-direction. In this case, when the sub-nozzles 21 are inserted into two nostrils, the coupling pipe 41 may interfere with, for example, the upper lip and may not allow insertion of the sub-nozzles 21 at an appropriate angle with respect to the nostrils.

In contrast, in the above structure, the coupling pipe 41 overlaps a portion of each receiver 45, rather than the entire portion of each receiver 45, in a plan view of the adapter 4. The coupling pipe 41 can thus have a decreased size in Y-direction while having a cross section that is in the shape of a perfect circle or substantially a perfect circle along its entire length. The sprayer 1 can thus reduce leakage of the liquid from the connection between the coupling pipe 41 and the syringe 9 and also allow the sub-nozzles 21 to be inserted into the nostrils at an appropriate angle. The coupling pipe 41 having a small diameter can reduce the amount of liquid remaining in the coupling pipe 41 to be discarded after spraying. The sprayer 1 can thus increase the use efficiency of the liquid.

In the sprayer 1 according to the present embodiment, the coupling pipe 41 includes the large cross-sectional portion 412 nearer the coupling end 41a than the holes 451 in the axial direction, and the small cross-sectional portion 413 nearer the coupling end 41a than the large cross-sectional portion 412 in the axial direction. The small cross-sectional portion 413 has a smaller cross section than the large cross-sectional portion 412 in the axial direction. The sprayer 1 further includes the stopper 5 in the internal space of the coupling pipe 41. The stopper 5 is in contact with the inner surface 41c of the coupling pipe 41 in a liquid tight manner in the small cross-sectional portion 413 and defines a clearance with the inner surface 41c of the coupling pipe 41 in the large cross-sectional portion 412. The stopper 5 slides from the small cross-sectional portion 413 to the large cross-sectional portion 412 when pressed by the liquid in a direction from the connection end 41a in the axial direction.

In this structure, once the stopper 5 starts sliding, the stopper 5 is pressed by the plunger 93 through the liquid and is also pressed under the repulsive force of the compressed air. The stopper 5 thus accelerates suddenly. In other words, this structure takes less time from when the stopper 5 and the plunger 93 pressing the stopper 5 start moving to when the liquid starts being sprayed. This allows a sufficient force for the stopper 5 to start sliding to be applied to the liquid at the start of spraying. In other words, a sufficient force for spraying can be applied to the liquid at the start of spraying, thus allowing the liquid to be sprayed stably.

In the sprayer 1 according to the present embodiment, the coupling pipe 41 includes the expanded portion 414 between the large cross-sectional portion 412 and the small cross-sectional portion 413. The expanded portion 414 has a cross section gradually increasing toward the small cross-sectional portion 413 in the axial direction.

In this structure, when the liquid stopper 52 reaches the expanded portion 414 in the coupling pipe 41, the stopper 5 is pressed toward the connection end 41b of the coupling pipe 41 by the contact between the liquid stopper 52 and the inclined inner surface 41c of the coupling pipe 41. This further accelerates the stopper 5, thus further shortening the time from when the stopper 5 and the plunger 93 start moving to when the liquid starts being sprayed. A sufficient force for spraying can thus be applied to the liquid at the start of spraying, thus allowing the liquid to be sprayed more stably.

In the sprayer 1 according to the present embodiment, each of the nozzle 2 and the adapter 4 is symmetric about the axis of the coupling pipe 41.

In this structure, the nozzle 2 and the adapter 4 symmetric about the axis of the coupling pipe 41 allow the two flow channels to be symmetric. This allows the liquid to be sprayed from the two nozzle bores 23 in the same manner and thus sprayed into both nostrils evenly.

### (Modifications)

The embodiment of the present disclosure described above is a mere example of the present disclosure. The exemplary embodiment described above may be modified or altered variously. For example, the embodiment may be modified in the forms described below. The modifications described below may be combined as appropriate.

### (First Modification)

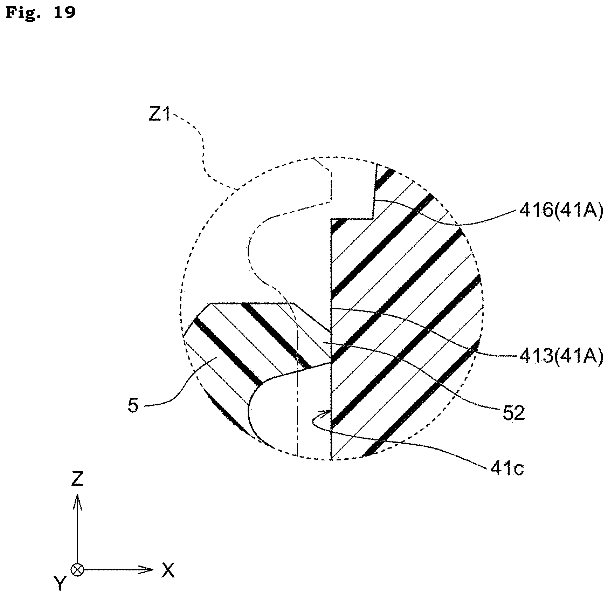

Fig. 19 is a partially enlarged view of a sprayer according to a first modification of the embodiment of the present disclosure corresponding to Fig. 11. Unlike the coupling pipe 41 shown in Fig. 11, a coupling pipe 41A shown in Fig. 19 includes no large cross-sectional portion 412 and no expanded portion 414. As shown in Fig. 19, the small cross-sectional portion 413 and the recess 416 are continuous with each other in the axial direction of the imaginary axis C2. For example, the boundary between the small cross-sectional portion 413 and the recess 416 is stepped. As indicated by the two-dot-dash line in Fig. 19, when reaching the recess 416 on the coupling pipe 41A, the liquid stopper 52 in the stopper 5 separates from the inner surface 41c of the coupling pipe 41A. This allows the compressed liquid to flow toward, in the coupling pipe 41A, the connection end 41b (upward in Fig. 19) beyond the liquid stopper 52 in the stopper 5.

This structure also shortens the time from when the stopper 5 and the plunger 93 start moving to when the liquid starts being sprayed as in the embodiment described above. A sufficient force for spraying can thus be applied to the liquid at the start of spraying, thus allowing the liquid to be sprayed more stably. This structure includes no expanded portion 414 having a surface inclined with respect to the imaginary axis C2. The adapter 4 may thus be formed using a simple-shaped mold by injection molding.

### (Second Modification)

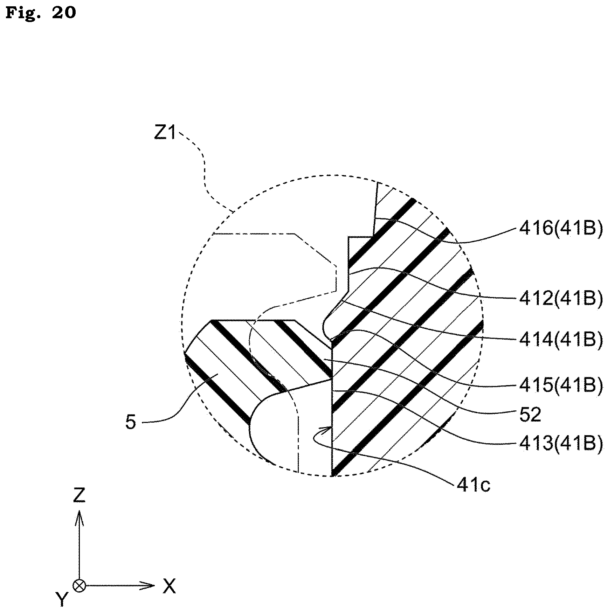

Fig. 20 is a partially enlarged view of a sprayer according to a second modification of the embodiment of the present disclosure corresponding to Fig. 11. Unlike the coupling pipe 41 shown in Fig. 11, a coupling pipe 41B shown in Fig. 20 further includes a reduced portion 415. The reduced portion 415 is between the expanded portion 414 and the small cross-sectional portion 413. The reduced portion 415 has a cross section gradually decreasing toward the expanded portion 414 along the corresponding imaginary axis C1 in the axial direction of the coupling pipe 41B.

In this structure, the coupling pipe 41B including the reduced portion 415 has a larger difference in its cross-sectional area in the expanded portion 414. The stopper 5 can thus be pressed harder toward the connection end 41b of the coupling pipe 41B when the liquid stopper 52 and the inclined inner surface 41c of the coupling pipe 41B come in contact with each other. This further accelerates the stopper 5, thus further shortening the time from when the stopper 5 and the plunger 93 start moving to when the liquid starts being sprayed. A sufficient force for spraying can thus be applied to the liquid at the start of spraying, thus allowing the liquid to be sprayed more stably.

### (Third Modification)

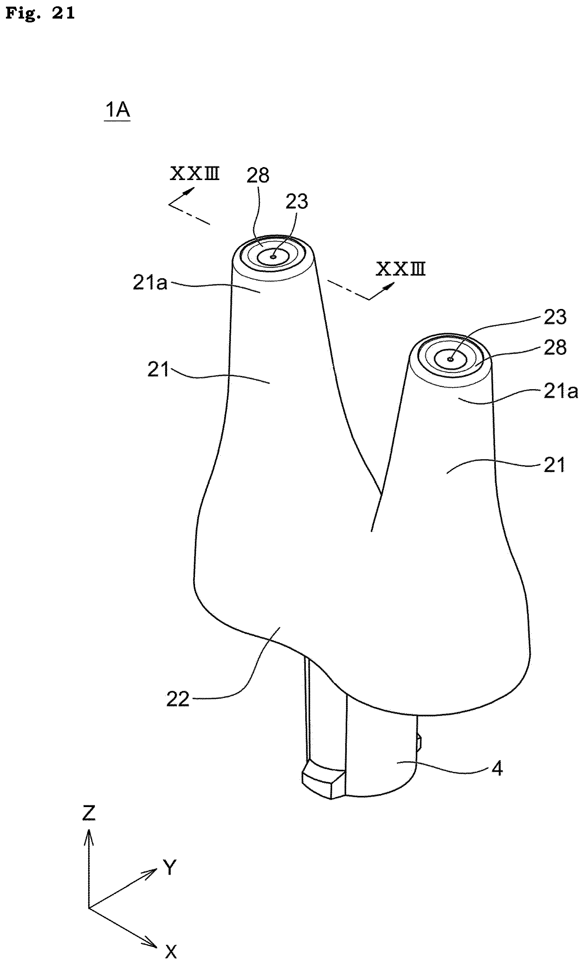

Fig. 21 is a perspective view of a sprayer 1A according to a third modification of the embodiment of the present disclosure. Unlike the sprayer 1 shown in Fig. 3, the sprayer 1A shown in Fig. 21 includes open distal ends 21a of the sub-nozzles 21. A chip 28 is located adjacent to the distal end 21a of each sub-nozzle 21.

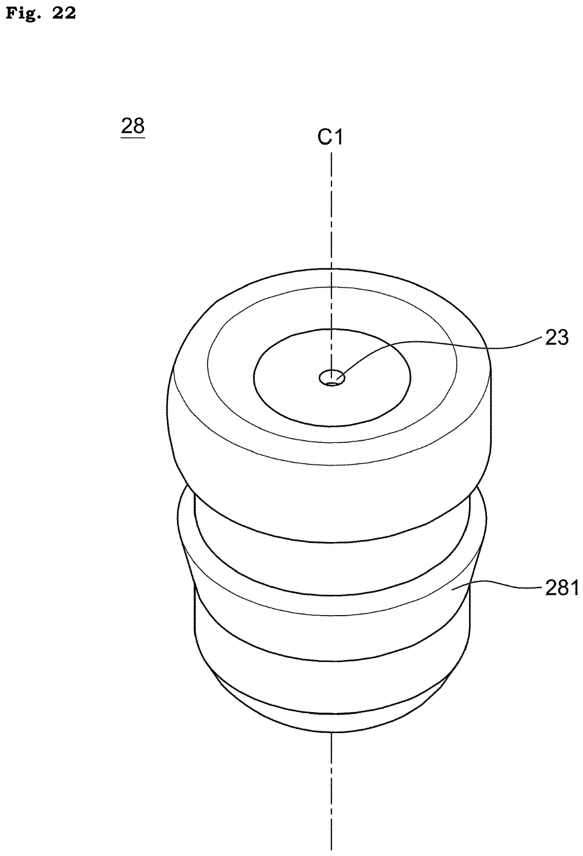

Fig. 22 is a perspective view of the chip 28 in the sprayer 1A in Fig. 21. For ease of explanation, each rod 3 described below is located in the internal space of the corresponding sub-nozzle 21. As shown in Fig. 22, the chip 28 is substantially cylindrical and extends in a direction in which the corresponding sub-nozzle 21 extends, or in other words, in the axial direction of the corresponding imaginary axis C1. The chip 28 includes an annular protrusion 281 in its middle portion in the axial direction of the corresponding imaginary axis C1. The protrusion 281 protrudes outward and extends in the circumferential direction of the corresponding imaginary axis C1. The protrusion 281 is used to attach the chip 28 to the corresponding sub-nozzle 21.

The chip 28 has a nozzle bore 23 in its end nearer the distal end 21a of the sub-nozzle 21 in the axial direction of the imaginary axis C1. The nozzle bore 23 may have the same shape and size as, for example, the nozzle bores 23 (refer to Fig. 4) in the embodiment described above.

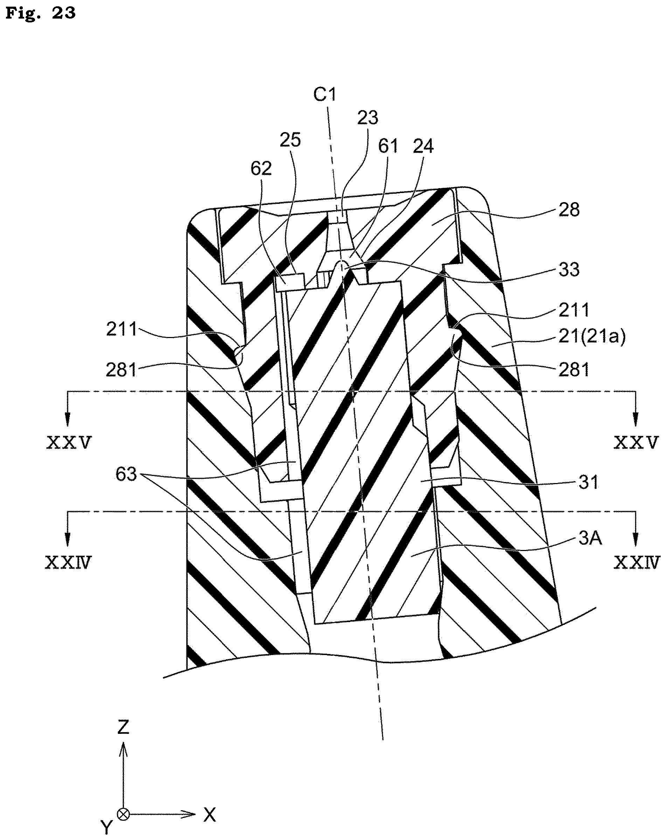

Fig. 23 is a cross-sectional view of the sprayer 1A in Fig. 21 taken along line XXIII-XXIII. More specifically, Fig. 23 is a partial cross-sectional view of the distal end 21a of one sub-nozzle 21 taken along a ZX plane as viewed in Y-direction. As shown in Fig. 23, the sub-nozzle 21 has an annular groove 211 on its inner side surface 21c. The chip 28 is placed through the distal end 21a of the sub-nozzle 21. The protrusion 281 on the chip 28 is fitted into the groove 211 on the sub-nozzle 21, thus allowing the chip 28 to be attached to the sub-nozzle 21.

The chip 28 attached to the sub-nozzle 21 receives a rod 3Ain its internal space. For example, as shown in Fig. 23, the chip 28 may receive a part of the rod 3A in its internal space.

The chip 28 has, on its inner surface, a recess 24 and three grooves 25 connecting with the recess 24. Fig. 23 shows one of the three grooves 25. The rod 3A is in contact with the inner surface of the chip 28 in the axial direction of the imaginary axis C1. This defines a spray chamber 61 between the recess 24 on the chip 28 and the rod 3A. The spray chamber 61 connects with the nozzle bore 23. The guide paths 62 are defined between the grooves 25 on the chip 28 and the rod 3A.

The rod 3A includes a projection 33 protruding toward the nozzle bore 23 along the imaginary axis C1. The projection 33 allows the liquid entering the spray chamber 61 from the guide paths 62 to easily flow spirally about the projection 33.

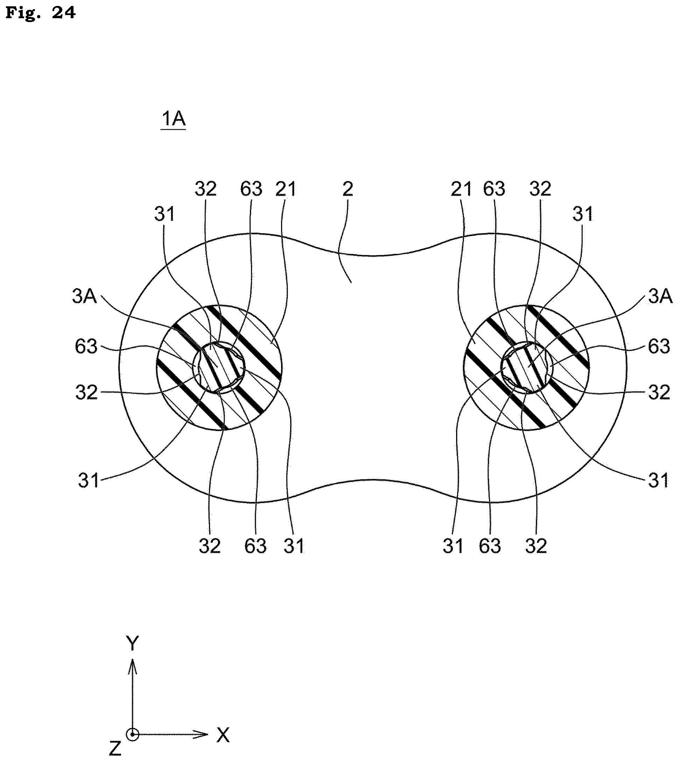

The rod 3A includes raised portions 31 extending from the lower end to the middle portion of the rod 3Ain the axial direction of the imaginary axis C1 and protruding outward. Fig. 24 is a cross-sectional view of the sprayer 1A in Fig. 23 taken along line XXIV-XXIV. As shown in Fig. 24, each rod 3A includes, for example, three raised portions 31. Each rod 3A has grooves 32 each between adjacent raised portions 31. The grooves 32 are recessed from the raised portions 31 and extend in the axial direction of the imaginary axes C1.

The raised portions 31 in each rod 3A are in contact with the inner side surface 21c of the corresponding sub-nozzle 21. The inner side surface 21c of each sub-nozzle 21 and the grooves 32 on the corresponding rod 3A define parts of the rod paths 63 between them.

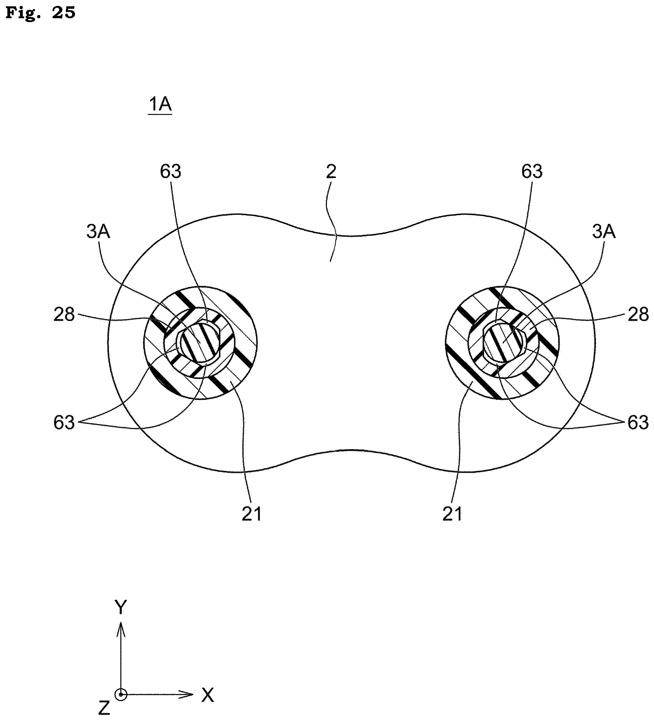

Fig. 25 is a cross-sectional view of the sprayer 1A in Fig. 23 taken along line XXV-XXV. As shown in Fig. 25, each rod 3A is partly in contact with the inner surface of the corresponding chip 28. In portions in which the rod 3A and the chip 28 are not in contact with each other, the rod 3A and the chip 28 define parts of the rod paths 63 between them. In the present modification as well, the rod paths 63 include portions with their channel cross sections smaller than the channel cross sections of the chambers 64.

The sprayer according to one or more embodiments of the present disclosure can reduce liquid leakage, and the technique is thus useful for various sprayers.

- 1, 1A: sprayer
- 2: nozzle
- 21: sub-nozzle
- 21a: distal end
- 21b: basal end
- 21d: outer surface
- 23: nozzle bore
- 4: adapter
- 41, 41A, 41B: coupling pipe
- 41a: coupling end
- 42: core
- 42a: distal end
- 42b: basal end
- 42c: outer side surface
- 43: circumferential wall
- 43a: facing surface
- 45: receiver
- 451: hole
- 65: core path
- 66: contact
- 9: syringe

## Claims

1. A sprayer (1) comprising
a nozzle (2) insertable into a nostril; and
an adapter (4) to connect the nozzle and a container containing liquid,
wherein the nozzle comprises at least one sub-nozzle (21) having a distal end (21a) and a basal end (21b),
the distal end comprises a nozzle bore (23) and is insertable into the nostril,
the adapter comprises a coupling pipe (41), two cores (42), and receivers (45) being U-shaped and annular,
the coupling pipe comprises a coupling end (41a) connectable to the container to allow fluid passage,
each of the two cores extends in an axial direction being an extending direction of the coupling pipe and comprises an axis different from an axis (C1) of the coupling pipe (C2) in a radial direction intersecting with the axial direction,
the two cores are adjacent to each other,
each of the receivers is at an outer side surface (42c) and a basal end (42b) of a corresponding core of the two cores and is open toward a distal end of the corresponding core,
each of the receivers comprises a hole (451) connecting with the coupling pipe to allow fluid passage,
the nozzle is attached to the adapter with a core of the two cores received in an internal space of the at least one sub-nozzle and the basal end of the at least one sub-nozzle received in a corresponding receiver of the receivers,
the at least one sub-nozzle and the core define a core path (65) in between,
the core path connects with the coupling pipe through the hole to allow fluid passage,
the core path is at least a part of a flow channel extending from the hole to the nozzle bore,
in an attachment state of the nozzle attached to the adapter, an outer surface (21d) of the at least one sub-nozzle and a facing surface (43a) of the corresponding receiver facing the outer surface are in contact with each other in a liquid tight manner and divide the core path from outside the core path, and
in the attachment state, a contact (66) between the outer surface of the at least one sub-nozzle and the facing surface of the corresponding receiver is in a shape of a perfect circle or substantially a perfect circle in a cross section intersecting with the axial direction.

2. A sprayer according to claim 1, wherein the contact is between the coupling pipe and the nozzle bore in the axial direction.

3. A sprayer according to claim 1 or 2, wherein
in the attachment state, the facing surface of each of the receivers is inclined away from the outer side surface of the corresponding core toward an opening in the receiver in the axial direction, and
the outer surface of the at least one sub-nozzle and the facing surface of the corresponding receiver are linearly in contact with each other at the contact.

4. A sprayer according to any one of claims 1-3, wherein
each of the receivers comprises an annular projection protruding outward from an outer surface being a surface opposite to the facing surface,
the nozzle comprises a protrusion protruding toward the facing surface of a corresponding receiver of the receivers in the attachment state, and
in the attachment state, the protrusion on the nozzle is located farther from the nozzle bore than the annular projection on each of the receivers in the axial direction and is in contact with the annular projection on the corresponding receiver.

5. A sprayer according to any one of claims 1-4, wherein
the nozzle comprises two sub-nozzles,
in the attachment state, each of the cores is received in the internal space of a corresponding sub-nozzle of the two sub-nozzles, and
in the attachment state, the contact between the outer surface of each of the two sub-nozzles and the facing surface of the corresponding receiver is in a shape of a perfect circle or substantially a perfect circle in a cross section intersecting with the axial direction.

6. A sprayer according to claim 5, wherein each of the two cores comprises an edge defined by the basal end of the core in a plan view of the adapter.

7. A sprayer according to claim 5 or 6, wherein
each of the two sub-nozzles comprises, in the internal space of the sub-nozzle and between the distal end of the sub-nozzle and the distal end of a corresponding core of the two cores, a chamber being at least a part of the flow channel, and
the chamber comprises a larger channel cross section than the core path in the flow channel.

8. A sprayer according to claim 7, further comprising rods each in the internal space of a corresponding sub-nozzle of the two sub-nozzles, each of the rods being between the distal end of the corresponding sub-nozzle and the distal end of the corresponding core,
wherein the chamber is between each of the rods and the distal end of the corresponding sub-nozzle, and
each of the rods and an inner side surface of the corresponding sub-nozzle define a rod path in between.

9. A sprayer according to claim 8, wherein the rod path comprises a smaller channel cross section than the chamber in the flow channel.

10. A sprayer according to claim 8 or 9, wherein
each of the two sub-nozzles comprises a projection protruding inward from the corresponding inner side surface,
the corresponding rod comprises a raised portion protruding outward,
in the attachment state, the projection on each of the two sub-nozzles is located farther from the corresponding nozzle bore than the raised portion in the corresponding rod in the axial direction and is in contact with the raised portion in the corresponding rod,
each of the rods is in contact with the distal end of the corresponding sub-nozzle, and
each of the rods and the distal end of the corresponding sub-nozzle define a guide path in between, and the guide path extends in a direction intersecting with the axial direction and connects the rod path and the nozzle bore to allow fluid passage.

11. A sprayer according to any one of claims 1-10, wherein the core path comprises a smaller channel cross section than the hole in the flow channel.

12. A sprayer according to any one of claims 1-11, wherein each of the receivers comprises a portion overlapping the coupling pipe and a portion not overlapping the coupling pipe in a plan view of the adapter.

13. A sprayer according to any one of claims 1-12, wherein
the coupling pipe comprises:
a large cross-sectional portion nearer the coupling end than the hole in the axial direction; and
a small cross-sectional portion nearer the coupling end than the large cross-sectional portion in the axial direction and having a smaller cross section than the large cross-sectional portion in the axial direction,
the sprayer further comprises a stopper in an internal space of the coupling pipe,
the stopper is in contact with an inner surface of the coupling pipe in a liquid tight manner in the small cross-sectional portion and defines a clearance with the inner surface of the coupling pipe in the large cross-sectional portion, and
the stopper is configured to slide from the small cross-sectional portion to the large cross-sectional portion when pressed by the liquid in a direction from the connection end in the axial direction.

14. A sprayer according to claim 13, wherein the coupling pipe comprises an expanded portion between the large cross-sectional portion and the small cross-sectional portion, and the expanded portion comprises a cross section gradually increasing toward the large cross-sectional portion in the axial direction.

15. A sprayer according to claim 14, wherein the coupling pipe comprises a reduced portion between the expanded portion and the small cross-sectional portion, and the reduced portion comprises a cross section gradually decreasing away from the small cross-sectional portion in the axial direction.

## Patentansprüche

1. Sprühgerät (1), umfassend:
eine Düse (2), die in ein Nasenloch einsetzbar ist; und
einen Adapter (4), um die Düse und einen Behälter, der Flüssigkeit enthält, zu verbinden,
wobei
die Düse zumindest eine Teildüse (21) mit einem distalen Ende (21a) und einem basalen Ende (21b) umfasst,
das distale Ende eine Düsenbohrung (23) umfasst und in das Nasenloch einführbar ist,
der Adapter ein Kopplungsrohr (41), zwei Kerne (42) und Aufnahmen (45) umfasst, die U-förmig und ringförmig sind,
das Kopplungsrohr ein Kopplungsende (41a) umfasst, das mit dem Behälter verbindbar ist, um Fluiddurchgang zu ermöglichen,
sich jeder der zwei Kerne in einer axialen Richtung erstreckt, die eine Erstreckungsrichtung des Kopplungsrohrs ist, und eine Achse umfasst, die sich von einer Achse (C1) des Kopplungsrohrs (C2) in einer radialen Richtung, welche die axiale Richtung schneidet, unterscheidet,
die zwei Kerne benachbart zueinander sind,
jede der Aufnahmen an einer Außenseitenfläche (42c) und einem basalen Ende (42b) eines entsprechenden Kerns der zwei Kerne ist und zu einem distalen Ende des entsprechenden Kerns offen ist,
jede der Aufnahmen ein Loch (451) umfasst, das sich mit dem Kopplungsrohr verbindet, um Fluiddurchgang zu ermöglichen,
die Düse an dem Adapter angebracht ist, wobei ein Kern der zwei Kerne in einem Innenraum der zumindest einen Teildüse aufgenommen ist und das basale Ende der zumindest einen Teildüse in einer entsprechenden Aufnahme der Aufnahmen aufgenommen ist,
die zumindest eine Teildüse und der Kern einen Kernweg (65) dazwischen definieren,
sich der Kernweg mit dem Kopplungsrohr durch das Loch verbindet, um Fluiddurchgang zu ermöglichen,
der Kernweg zumindest ein Teil eines Flusskanals ist, der sich von dem Loch zu der Düsenbohrung erstreckt,
in einem Anbringungszustand der Düse, die an dem Adapter angebracht ist, eine Außenfläche (21d) der zumindest einen Teildüse und eine zugewandte Fläche (43a) der entsprechenden Aufnahme, die der Außenfläche zugewandt ist, auf eine flüssigkeitsdichte Weise in Kontakt miteinander sind und den Kernweg von außerhalb des Kernwegs unterteilen und
in dem Anbringungszustand ein Kontakt (66) zwischen der Außenfläche der zumindest einen Teildüse und der zugewandten Fläche der entsprechenden Aufnahme in einer Form eines perfekten Kreises oder im Wesentlichen eines perfekten Kreises in einem Querschnitt, der sich mit der axialen Richtung schneidet, ist.

2. Sprühgerät nach Anspruch 1, wobei der Kontakt zwischen dem Kopplungsrohr und der Düsenbohrung in der axialen Richtung ist.

3. Sprühgerät nach Anspruch 1 oder 2, wobei
in dem Anbringungszustand die zugewandte Fläche von jeder der Aufnahmen weg von der Außenseitenfläche des entsprechenden Kerns zu einer Öffnung in der Aufnahme in der axialen Richtung geneigt ist und
die Außenfläche der zumindest einen Teildüse und die zugewandte Fläche der entsprechenden Aufnahme linear in Kontakt miteinander an dem Kontakt sind.

4. Sprühgerät nach einem der Ansprüche 1-3, wobei
jede der Aufnahmen einen ringförmigen Überstand umfasst, der von einer Außenfläche, die eine Fläche gegenüber der zugewandten Fläche ist, nach außen vorspringt,
die Düse einen Vorsprung umfasst, der zu der zugewandten Fläche einer entsprechenden Aufnahme der Aufnahmen in dem Anbringungszustand vorspringt, und
sich in dem Anbringungszustand der Vorsprung an der Düse weiter von der Düsenbohrung als der ringförmige Überstand an jeder der Aufnahmen in der axialen Richtung befindet und in Kontakt mit dem ringförmigen Überstand an der entsprechenden Aufnahme ist.

5. Sprühgerät nach einem der Ansprüche 1-4, wobei
die Düse zwei Teildüsen umfasst,
in dem Anbringungszustand jeder der Kerne in dem Innenraum einer entsprechenden Teildüse der zwei Teildüsen aufgenommen ist und
in dem Anbringungszustand der Kontakt zwischen der Außenfläche von jeder der zwei Teildüsen und der zugewandten Fläche der entsprechenden Aufnahme in einer Form eines perfekten Kreises oder im Wesentlichen eines perfekten Kreises in einem Querschnitt, der sich mit der axialen Richtung schneidet, ist.

6. Sprühgerät nach Anspruch 5, wobei jeder der zwei Kerne eine Kante umfasst, die durch das basale Ende des Kerns in einer Draufsicht auf den Adapter definiert ist.

7. Sprühgerät nach Anspruch 5 oder 6, wobei
jede der zwei Teildüsen in dem Innenraum der Teildüse und zwischen dem distalen Ende der Teildüse und dem distalen Ende eines entsprechenden Kerns der zwei Kerne eine Kammer umfasst, die zumindest ein Teil des Flusskanals ist, und
die Kammer einen größeren Kanalquerschnitt als der Kernweg in dem Flusskanal umfasst.

8. Sprühgerät nach Anspruch 7, ferner umfassend Stäbe jeweils in dem Innenraum einer entsprechenden Teildüse der zwei Teildüsen, wobei jeder der Stäbe zwischen dem distalen Ende der entsprechenden Teildüse und dem distalen Ende des entsprechenden Kerns ist,
wobei die Kammer zwischen jedem der Stäbe und dem distalen Ende der entsprechenden Teildüse ist und
jeder der Stäbe und eine Innenseitenfläche der entsprechenden Teildüse einen Stabweg dazwischen definieren.

9. Sprühgerät nach Anspruch 8, wobei der Stabweg einen kleineren Kanalquerschnitt als die Kammer in dem Flusskanal umfasst.

10. Sprühgerät nach Anspruch 8 oder 9, wobei
jede der zwei Teildüsen einen Überstand umfasst, der von der entsprechenden Innenseitenfläche nach innen vorspringt,
der entsprechende Stab einen erhöhten Abschnitt umfasst, der nach außen vorspringt,
sich in dem Anbringungszustand der Überstand an jeder der zwei Teildüsen weiter von der entsprechenden Düsenbohrung als der erhöhte Abschnitt in dem entsprechenden Stab in der axialen Richtung befindet und in Kontakt mit dem erhöhten Abschnitt in dem entsprechenden Stab ist,
jeder der Stäbe in Kontakt mit dem distalen Ende der entsprechenden Teildüse ist und
jeder der Stäbe und das distale Ende der entsprechenden Teildüse einen Führungsweg dazwischen definieren und sich der Führungsweg in einer Richtung erstreckt, welche die axiale Richtung schneidet, und den Stabweg und die Düsenbohrung verbindet, um Fluiddurchgang zu ermöglichen.

11. Sprühgerät nach einem der Ansprüche 1-10, wobei der Kernweg einen kleineren Kanalquerschnitt als das Loch in dem Flusskanal umfasst.

12. Sprühgerät nach einem der Ansprüche 1-11, wobei in einer Draufsicht auf den Adapter jede der Aufnahmen einen Abschnitt, der das Kopplungsrohr überlappt, und einen Abschnitt, der das Kopplungsrohr nicht überlappt, umfasst.

13. Sprühgerät nach einem der Ansprüche 1-12, wobei
das Kopplungsrohr Folgendes umfasst:
einen großen Querschnittsabschnitt näher an dem Kopplungsende als das Loch in der axialen Richtung; und
einen kleinen Querschnittsabschnitt näher an dem Kopplungsende als der große Querschnittsabschnitt in der axialen Richtung und mit einem kleineren Querschnitt als der große Querschnittsabschnitt in der axialen Richtung,
das Sprühgerät ferner einen Anschlag in einem Innenraum des Kopplungsrohrs umfasst,
der Anschlag in Kontakt mit einer Innenfläche des Kopplungsrohrs auf eine flüssigkeitsdichte Weise in dem kleinen Querschnittsabschnitt ist und einen Freiraum mit der Innenfläche des Kopplungsrohrs in dem großen Querschnittsabschnitt definiert und
der Anschlag dazu konfiguriert ist, von dem kleinen Querschnittsabschnitt zu dem großen Querschnittsabschnitt zu gleiten, wenn er durch die Flüssigkeit in einer Richtung von dem Verbindungsende in der axialen Richtung gedrückt wird.

14. Sprühgerät nach Anspruch 13, wobei das Kopplungsrohr einen erweiterten Abschnitt zwischen dem großen Querschnittsabschnitt und dem kleinen Querschnittsabschnitt umfasst und der erweiterte Abschnitt einen Querschnitt umfasst, der allmählich zu dem großen Querschnittsabschnitt in der axialen Richtung zunimmt.

15. Sprühgerät nach Anspruch 14, wobei das Kopplungsrohr einen reduzierten Abschnitt zwischen dem erweiterten Abschnitt und dem kleinen Querschnittsabschnitt umfasst und der reduzierte Abschnitt einen Querschnitt umfasst, der allmählich weg von dem kleinen Querschnittsabschnitt in der axialen Richtung abnimmt.

## Revendications

1. Pulvérisateur (1) comprenant :
une buse (2) pouvant être insérée dans une narine ; et
un adaptateur (4)
pour raccorder la buse et un récipient contenant du liquide,
ladite buse comprenant au moins une sous-buse (21) possédant une extrémité distale (21a) et une extrémité basale (21b),
ladite extrémité distale comprenant un alésage (23) de buse et pouvant être insérée dans la narine,
ledit adaptateur comprenant un tuyau de couplage (41), deux noyaux (42) et des récepteurs (45) en forme de U et annulaires,
ledit tuyau de couplage comprenant une extrémité de couplage (41a) pouvant être raccordée au récipient pour permettre le passage du fluide,
chacun des deux noyaux s'étendant dans une direction axiale qui est une direction d'extension du tuyau de couplage et comprenant un axe différent d'un axe (C1) du tuyau de couplage (C2) dans une direction radiale coupant la direction axiale,
lesdits deux noyaux étant adjacents l'un à l'autre,
chacun des récepteurs se trouvant au niveau d'une surface latérale externe (42c) et d'une extrémité basale (42b) d'un noyau correspondant des deux noyaux et étant ouvert vers une extrémité distale du noyau correspondant,
chacun des récepteurs comprenant un trou (451) se raccordant au tuyau de couplage pour permettre le passage du fluide,
ladite buse étant fixée à l'adaptateur avec un noyau des deux noyaux reçu dans un espace intérieur de l'au moins une sous-buse et l'extrémité de base de l'au moins une sous-buse reçue dans un récepteur correspondant des récepteurs,
ladite au moins une sous-buse et ledit noyau définissant un trajet de noyau (65) entre eux,
ledit trajet de noyau se raccordant au tuyau de couplage à travers le trou pour permettre le passage du fluide,
ledit trajet de noyau faisant au moins partie d'un canal d'écoulement s'étendant à partir du trou jusqu'à l'alésage de buse,
dans un état de fixation de la buse fixée à l'adaptateur, une surface externe (21d) de l'au moins une sous-buse et une surface en regard (43a) du récepteur correspondant faisant face à la surface externe étant en contact l'une avec l'autre de manière étanche aux liquides et divisant le trajet de noyau de l'extérieur du trajet de noyau, et
dans l'état de fixation, un contact (66) entre la surface externe de l'au moins une sous-buse et la surface en regard du récepteur correspondant possédant la forme d'un cercle parfait ou
sensiblement d'un cercle parfait dans une section transversale coupant la direction axiale.

2. Pulvérisateur selon la revendication 1, ledit contact étant entre le tuyau de couplage et l'alésage de buse dans la direction axiale.

3. Pulvérisateur selon la revendication 1 ou 2,
dans l'état de fixation, ladite surface en regard de chacun des récepteurs étant inclinée en s'éloignant de la surface latérale externe du noyau correspondant vers une ouverture dans le récepteur dans la direction axiale, et
ladite surface externe de l'au moins une sous-buse et ladite surface en regard du récepteur correspondant étant en contact linéaire l'une avec l'autre au niveau du contact.

4. Pulvérisateur selon l'une quelconque des revendications 1 à 3,
chacun des récepteurs comprenant une saillie annulaire faisant saillie vers l'extérieur à partir d'une surface externe qui est une surface opposée à la surface en regard,
ladite buse comprenant une saillie faisant saillie vers la surface en regard d'un récepteur correspondant des récepteurs dans l'état de fixation, et
dans l'état de fixation, ladite saillie sur la buse étant située plus loin de l'alésage de la buse que la saillie annulaire sur chacun des récepteurs dans la direction axiale et étant en contact avec la saillie annulaire sur le récepteur correspondant.

5. Pulvérisateur selon l'une quelconque des revendications 1 à 4,
ladite buse comprenant deux sous-buses,
dans l'état de fixation, chacun des noyaux étant reçu dans l'espace intérieur d'une sous-buse correspondante des deux sous-buses, et
dans l'état de fixation, le contact entre la surface externe de chacune des deux sous-buses et la surface en regard du récepteur correspondant possédant la forme d'un cercle parfait ou possédant sensiblement la forme d'un cercle parfait dans une section transversale coupant la direction axiale.

6. Pulvérisateur selon la revendication 5, chacun des deux noyaux comprenant un bord défini par l'extrémité de base du noyau dans une vue en plan de l'adaptateur.

7. Pulvérisateur selon la revendication 5 ou 6,
chacune des deux sous-buses comprenant, dans l'espace intérieur de la sous-buse et entre l'extrémité distale de la sous-buse et l'extrémité distale d'un noyau correspondant des deux noyaux, une chambre faisant au moins partie du canal d'écoulement, et
ladite chambre comprenant une section transversale de canal plus grande que le trajet de noyau dans le canal d'écoulement.

8. Pulvérisateur selon la revendication 7, comprenant en outre des tiges chacune dans l'espace intérieur d'une sous-buse correspondante des deux sous-buses, chacune des tiges se trouvant entre l'extrémité distale de la sous-buse correspondante et l'extrémité distale du noyau correspondant,
ladite chambre se trouvant entre chacune des tiges et l'extrémité distale de la sous-buse correspondante, et
chacune des tiges et une surface latérale interne de la sous-buse correspondante définissant un trajet de tige entre elles.

9. Pulvérisateur selon la revendication 8, ledit trajet de tige comprenant une section transversale de canal plus petite que la chambre dans le canal d'écoulement.

10. Pulvérisateur selon la revendication 8 ou 9,
chacune des deux sous-buses comprenant une saillie faisant saillie vers l'intérieur à partir de la surface latérale interne correspondante,
ladite tige correspondante comprenant une partie surélevée faisant saillie vers l'extérieur,
dans l'état de fixation, ladite saillie sur chacune des deux sous-buses étant située plus loin de l'alésage de la buse correspondante que la partie surélevée dans la tige correspondante dans la direction axiale et étant en contact avec la partie surélevée dans la tige correspondante,
chacune des tiges étant en contact avec l'extrémité distale de la sous-buse correspondante, et
chacune des tiges et l'extrémité distale de trajet de guidage la sous-buse correspondante définissant un trajet de guidage entre elles, et ledit s'étendant dans une direction coupant la direction axiale et raccordant le trajet de tige et l'alésage de buse pour permettre le passage de fluide.

11. Pulvérisateur selon l'une quelconque des revendications 1 à 10, ledit trajet de noyau comprenant une section transversale de canal plus petite que le trou dans le canal d'écoulement.

12. Pulvérisateur selon l'une quelconque des revendications 1 à 11, chacun des récepteurs comprenant une partie chevauchant le tuyau de couplage et une partie ne chevauchant pas le tuyau de couplage dans une vue en plan de l'adaptateur.

13. Pulvérisateur selon l'une quelconque des revendications 1 à 12,
ledit tuyau de couplage comprenant :
une grande partie de section transversale plus proche de l'extrémité de couplage que le trou dans la direction axiale ; et
une petite partie de section transversale plus proche de l'extrémité de couplage que la grande partie de section transversale dans la direction axiale et possédant une section transversale plus petite que la grande partie de section transversale dans la direction axiale,
ledit pulvérisateur comprenant en outre un bouchon dans un espace intérieur du tuyau de couplage,
ledit bouchon étant en contact avec une surface interne du tuyau de couplage de manière étanche aux liquides dans la petite partie de section transversale et définissant un jeu avec la surface interne du tuyau de couplage dans la grande partie de section transversale, et
ledit bouchon étant conçu pour coulisser de la petite partie de section transversale à la grande partie de section transversale lorsqu'il est pressé par le liquide dans une direction allant de l'extrémité de connexion dans la direction axiale.

14. Pulvérisateur selon la revendication 13, ledit tuyau de couplage comprenant une partie étendue entre la grande partie de section transversale et la petite partie de section transversale, et ladite partie étendue comprenant une section transversale augmentant progressivement vers la grande partie de section transversale dans la direction axiale.

15. Pulvérisateur selon la revendication 14, ledit tuyau de couplage comprenant une partie réduite entre la partie étendue et la petite partie de section transversale, et ladite partie réduite comprenant une section transversale diminuant progressivement à partir de la petite partie de section transversale dans la direction axiale.
